(19)

**Europäisches Patentamt**
**European Patent Office**
**Office européen des brevets**

(11)  **EP 4 560 314 A1**

(12)  **EUROPEAN PATENT APPLICATION**

(43) Date of publication:
28.05.2025  Bulletin 2025/22

(51) International Patent Classification (IPC):
**G01N 33/483** (2006.01)    **G01N 21/00** (2006.01)

(21) Application number: 23212407.3

(52) Cooperative Patent Classification (CPC):
**G01N 33/4836**

(22) Date of filing: **27.11.2023**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Deutsches Zentrum für Neurodegenerative Erkrankungen e.V. (DZNE)**
**53127 Bonn (DE)**

(72) Inventors:
• **Amin, Hayder**
**01279 Dresden (DE)**
• **Kempermann, Gerd**
**01309 Dresden (DE)**

(74) Representative: **Krauss, Jan**
**SKM-IP PartGmbB**
**Oberanger 45**
**80331 München (DE)**

Remarks:
The application is published incomplete as filed (Rule 68(1) EPC).

(54)  **METHOD AND DEVICE FOR HIGH-RESOLUTION SPATIAL AND TEMPORAL MAPPING OF LARGE-SCALE ELECTROPHYSIOLOGICAL DYNAMICS AND TRANSCRIPTIONAL PROFILES WITHIN INTACT BRAIN TISSUES**

(57)  The present invention relates to an *ex vivo* method for mapping the spatiotemporal electrophysiological dynamics and spatial transcriptional profiles of cells in a functional neuronal cell assembly, comprising simultaneous recording and analysis of spatial data of molecular and electrical network activity down to the level of individual cells using high-density electrical biosensors, spatial transcriptomics, optical imaging, and advanced computational strategies. The invention further relates to methods for identifying the composition of a functional neuronal assembly, for monitoring the spatiotemporal electrophysiological dynamics and transcriptional profiles of cells in a functional neuronal cell assembly, for monitoring the cellular composition of a functional neuronal assembly, or for identifying a compound having an effect on the spatial electrophysiological and transcriptional dynamics and/or for identifying the composition of cells in a functional neuronal cell assembly based on the dynamics as detected, as well as devices and uses thereof. The invention allows for a better understanding of disease mechanisms, and to find therapeutic targets and to develop new drugs and treatments.

Figure 1

**EP 4 560 314 A1**

## Description

The present invention relates to an *ex vivo* method for mapping the spatiotemporal electrophysiological dynamics and spatial transcriptional profiles of cells in a functional neuronal cell assembly, comprising simultaneous recording and analysis of spatial data of molecular and electrical network activity down to the level of individual cells using high-density electrical biosensors, spatial transcriptomics, optical imaging, and advanced computational strategies. The invention further relates to methods for identifying the composition of a functional neuronal assembly, for monitoring the spatiotemporal electrophysiological dynamics and transcriptional profiles of cells in a functional neuronal cell assembly, for monitoring the cellular composition of a functional neuronal assembly, or for identifying a compound having an effect on the spatial electrophysiological and transcriptional dynamics and/or for identifying the composition of cells in a functional neuronal cell assembly based on the dynamics as detected, as well as devices and uses thereof. The invention allows for a better understanding of disease mechanisms, and to find therapeutic targets and to develop new drugs and treatments.

## Background of the invention

The brain evolved to process complex information robustly and efficiently to maintain homeostasis, navigate the world, make decisions, and perform higher cognitive functions (1). Understanding the complexity of the brain coherently from the molecular to system level requires integrating multimodal data from diverse spatiotemporal contexts.

At the core of this ambitious aim lies the integration of neuronal electrophysiological and molecular phenotypes at the network and cellular resolution, as these underlie physiological functions as well as neurodevelopmental and neurodegenerative diseases (2,3).

Methodological developments like patch-sequencing (patch-seq) have allowed single-cell transcriptomics and the morphologic reconstruction of individual neurons after electrophysiological recordings (4,5). Despite its extraordinary significance, patch-seq still suffers from very low throughput and shortcomings in resolving neuronal networks in medium to large spatial contexts.

The Electro-seq method combined flexible bioelectronics with *in situ* RNA sequencing to map electrical activity and gene expression but is only applicable to reductionist neuronal cultures (6).

WO 2023/091970A1 discloses a computer-implemented method to determine an omics profile of a cell using microscopy imaging data. The method combines microscopy imaging data of a cell or a population of cells; determining a targeted expression profile of a set of target genes from the microscopy imaging data, the target genes identifying a cell type or cell state of interest; and determining a single-cell omics profile for the cell or population of cells. The targeted expression profile may be a targeted spatial expression profile, the microscopy imaging data may be obtained from a label-free microscopy method, and the cell or population of cells may be a live cell or a population of live cells.

By profiling expression patterns of thousands of neuronal genes while preserving spatial tissue architecture, high-throughput spatially resolved transcriptomics (SRT) is offering unparalleled insights into the molecular diversity of brain regions of interest (7,8). However, the temporal resolution of SRT is limited, providing only single snapshots of the transcriptomic landscape. Spatiotemporal transcriptomics is only resolvable using multiple samples from different time points (9,10).

US 2022-0068438A1 relates to methods, systems and computer products for aligning single cell data with spatial data to generate spatial maps of cell types and gene expression at single cell resolution. Further described is mapping to common coordinate frameworks.

WO 2021/168455A1 relates to a spatial detection of a nucleic acid, such as a genomic DNA or an RNA transcript, in a cell comprised in a tissue sample. Disclosed are methods for detecting and/or analyzing nucleic acids, such as chromatin or RNA transcripts, so as to obtain spatial information about the localization, distribution or expression of genes in a tissue sample. **Disclosed is a process for performing "spatial transcriptomics" or "spatial genomics," which** enables the user to determine simultaneously the expression pattern, or the location/distribution pattern of the genes expressed, or genes or genomic loci present in a single cell while retaining information related to the spatial location of the cell within the tissue architecture.

Aqrawe Z, et al. (in: A simultaneous optical and electrical in-vitro neuronal recording system to evaluate microelectrode performance. PLoS One. 2020 Aug 20;15(8):e0237709. doi: 10.1371/journal.pone.0237709. PMID: 32817653; PMCID: PMC7440637) disclose a setup of a high spatiotemporal resolution, electrical recording system utilizing planar microelectrode arrays with simultaneous optical imaging suitable for evaluating microelectrode performance **with a proposed 'performance factor' metric. Neuronal activity** was simultaneously recorded using both electrical and optical techniques and this activity was confirmed via tetrodotoxin application to inhibit action potential firing.

Brain-on-chip technologies empowered by high-density complementary metal-oxide semiconductors (CMOS) biosensing microelectrode arrays (MEA) are now allowing the non-invasive, multi-site, long-term, and label-free simultaneous measurements of extracellular activity capturing both local field potentials and spiking activity from thousands of neurons with single-cell accuracy without disruption of cellular integrity (13-17).

Despite the above improvements, there is still an unmet need to provide new and effective approaches to analyze the spatial realm of complex neuronal network dynamics across different scales.

It is therefore an object of the present invention, to provide a platform that can simultaneously capture and integrate molecular and functional information across multiple spatiotemporal scales within intact brain tissue samples. This shall bridge the gap between molecular architecture and brain activity, offering insights into causal neuronal plasticity and connectivity dynamics. Other objects and advantages of the present invention will become apparent to the person of skill in the art upon studying the present disclosure further.

According to a first aspect thereof, the above object has been solved according to the present invention by a method for detecting the spatiotemporal electrophysiological dynamics and transcriptional profiles of functional neuronal cell assemblies comprising the steps of providing a functional neuronal cell assembly to be characterized, recording patterns of local field potentials (LFPs) of the neuronal cells in the assembly at high spatial and temporal resolution using a suitable electrode array, and obtaining spatial localization patterns of the LFPs of substantially the entire assembly or group of assembly within the intact tissue using optical imaging, optionally, cryopreserving substantially the entire assembly or tissue, performing optical imaging and spatially resolved transcriptomic analysis of the cells in the assembly using a second array, and processing the data as obtained comprising aligning the spatial localization patterns of the LFPs with the spatially resolved transcriptomic analysis, and thereby detecting the spatiotemporal electrophysiological dynamics and spatial transcriptional profiles of cells in the functional neuronal cell assembly.

The methods according to the present invention may be performed in vitro or in vivo.

Preferred is the method according to the present invention, wherein the functional neuronal cell assembly to be characterized is selected from a neuronal tissue, a brain slice, a sample of neurological cancer, a culture of interconnected brain cells, cultured neuronal differentiated iPSCs, spinal marrow, electrogenic tissues like the olfactory bulb and cardiac tissues, and parts or combinations thereof, in particular a neuronal tissue derived from a mammal having a behavioral condition or disorder, a mammal having a neurological condition or disorder, such as a neurodegenerative disorder or cancer, and/or a mammal being treated for one of these conditions or disorders.

Further preferred is the method according to the present invention, wherein processing the data and aligning the spatial localization patterns of the LFPs with the spatially resolved sequencing and transcriptomic analysis, comprises overlaying the optical images with the electrode array layout, and overlaying the optical images with the second array layout, and alignment of the two overlays comprising image resizing and rotation.

According to a second aspect thereof, the above object has been solved according to the present invention by a method for identifying the composition of a functional neuronal assembly, comprising performing the method according to the present invention, and further comprising the step of concluding on the composition of the functional neuronal cell assemblies based on the spatiotemporal electrophysiological dynamics and spatial transcriptional profiles of the cells as detected.

According to a third aspect thereof, the above object has been solved according to the present invention by a method for monitoring the spatiotemporal electrophysiological dynamics and spatial transcriptional profiles of functional neuronal cell assemblies comprising performing the method according to the present invention repeatedly at different time points, and monitoring the spatial electrophysiological and transcriptional information of the molecular neuronal cell assemblies based on comparing the spatiotemporal electrophysiological dynamics and transcriptional profiles of the cells as detected at different timepoints.

According to a fourth aspect thereof, the above object has been solved according to the present invention by a method for monitoring the cellular composition of a functional neuronal assembly, comprising performing the method according to the present invention repeatedly at different time points and monitoring the cellular composition of the functional neuronal cell assembly based on comparing the cellular compositions of the functional neuronal cell assembly as detected at different timepoints.

According to a fifth aspect thereof, the above object has been solved according to the present invention by a method for identifying a compound having an effect on the spatiotemporal electrophysiological and spatial transcriptional profile and/or composition of cells in a functional neuronal cell assembly comprising performing the method according to the present invention in the presence and absence of at least one candidate compound, wherein a difference of the spatiotemporal electrophysiological and transcriptional dynamics and/or composition of cells in the functional neuronal cell assembly in the presence and absence of the at least one candidate compound or when compared to a suitable control identifies a compound having an effect on the spatiotemporal electrophysiological dynamics and transcriptional profile and/or composition of cells in a functional neuronal cell assembly.

According to a sixth aspect thereof, the above object has been solved according to the present invention by a device for performing the method according to the present invention, in particular comprising the neuronal cell assembly to be analyzed, an electrode array suitable to record patterns of local field potentials (LFPs) of the assembly while performing optical imaging, a second array suitable to perform spatially resolved transcriptomic analysis of the cells in the assembly while performing optical imaging, and a computer program product for processing the data as obtained comprising aligning the spatial localization patterns of the LFPs with the spatially resolved sequencing and transcriptomic analysis, optionally with additional buffers, and instructions for use.

According to a seventh aspect thereof, the above object has been solved according to the present invention by the use of the device according to the present invention for detecting the spatiotemporal electrophysiological dynamics and transcriptional profile of cells in a functional neuronal cell assembly, for identifying the composition of a functional neuronal cell assembly, for monitoring the spatialtemporal electrophysiological and spatial transcriptional dynamics of cells in a functional neuronal cell assembly, for monitoring the cellular composition of a functional neuronal cell assembly, or for identifying a compound having an effect on the spatiotemporal electrophysiological dynamics and transcriptional profile and/or for identifying the composition of cells in a functional neuronal cell assembly.

Brain functions are executed through the joint action of large assemblies of neurons and gene networks that share basic organizational principles, and information processing across a wide range of spatial and temporal scales that evolve with experience and change in disease.

Therefore, decoding the spatiotemporal electrophysiological and spatial transcriptomics information in the same tissue while retaining the cell assembly position can specify spatiotemporal transcriptomic networks, profiles and/or landscapes in their real-time relation to connectivity and other multimodal data, quantify molecular dynamics in pseudotime based on the underlying firing information, deconvolute spatially resolved cell type compositions, and predict electrophysiological network features from transcriptomic profiles with high accuracy.

As mentioned above, according to a first aspect thereof, the present invention provides a method for detecting the spatiotemporal electrophysiological dynamics and spatial transcriptional profiles of cells in a functional neuronal cell assembly. The method comprises providing a functional neuronal cell assembly or assemblies to be characterized. Then, patterns of local field potentials (LFPs) of the neuronal cells in the assembly are recorded at high spatial and temporal resolution using a suitable electrode array, while obtaining spatial localization patterns of the LFPs of substantially the entire assembly using optical imaging. Then, optionally, substantially the entire assembly is cryopreserved and may be stored before further analysis. Subsequently, optical imaging and spatially resolved transcriptomic analysis of the cell assemblies are performed using a second suitable array. Local field potentials (LFPs) in brain slice recordings in the context of the present invention are electrical signals measured from a group of neurons (assemblies) in a slice of brain tissue. These signals reflect the combined activity of these neurons, providing insights into how they communicate and function together. The data as obtained is processed comprising aligning the spatial localization patterns of the patterns of neural activity recorded by the electrode array as LFP signals (herein "LFPs") with the spatially resolved sequencing and transcriptomic analysis, preferably using machine-learning algorithms, and therefore including the timing and topology of the activity of the assembly in one high-resolution representation. Thereby, the spatiotemporal electrophysiological dynamics and spatial transcriptional profiles of functional neuronal cell assemblies is detected.

In a set of specific embodiments of the invention, the inventors here provide a method that is in **preferred embodiments termed "MEA-seqX", which combines brain**-on-chip, bioimaging, and spatial sequencing technology within a cross-scale computational framework. MEA-seqX comprises i) the simultaneous recording of electrophysiological firing patterns from large cell assemblies in brain slices at high spatiotemporal resolution, and imaging of the entire circuit for spatial localization, ii) multiplexed spatial profiling of cellular transcriptomics from the same cell assemblies in the same neural circuit, and iii) specifying spatiotemporal transcriptomic networks in their real-time relation to connectivity and other multimodal data, quantifying molecular dynamics in pseudotime based on the underlying firing information, deconvoluting spatially resolved cell type compositions, and/or predicting electrophysiological network features from transcriptomic profiles with high accuracy, using machine-learning algorithms and preserving time and topology in one high-resolution representation.

In general, the method according to the present invention can be used in many different analysis scenarios with respect to functional neuronal cell assemblies. In the context of the present **invention, the term "functional neuronal cell assemblies" or "neuronal assemblies" or "cell assemblies" shall refer to** a group of neurons that become activated and function together as a unit in response to specific types of stimulation or during particular behavioral processes. This concept is fundamental in understanding how the brain encodes and processes information, (see also, for example, Holtmaat, A., Caroni, P. Functional and structural underpinnings of neuronal assembly formation in learning. Nat Neurosci 19, 1553-1562 (2016). https://doi.org/10.1038/nn.4418, and Umbach, G., Tan, R., Jacobs, J. et al. Flexibility of functional neuronal assemblies supports human memory. Nat Commun 13, 6162 (2022). https://doi.org/10.1038/s41467-022-33587-0). For the purposes of the method according to the present invention, the neuronal cell assemblies need to remain essentially functional *in vitro,* i.e. need to exhibit a co-firing activity that essentially reflects their function *in vivo.*

The functional neuronal cell assemblies to be characterized may be selected from a neuronal tissue (assembly), a brain slice, a sample of neurological cancer, a culture of interconnected brain cells, cultured neuronal differentiated iPSCs, spinal marrow, electrogenic tissues like the olfactory bulb and cardiac tissues, and parts or combinations thereof. Particularly preferred is a neuronal tissue (assembly) derived from a mammal having a behavioral condition or disorder, a mammal having a neurological condition or disorder, such as a neurodegenerative disorder or cancer, and/or a mammal being treated for one of these conditions or disorders.

Then, patterns of local field potentials (LFPs) of the neuronal cells in the assembly are recorded at high spatial and temporal resolution using a suitable electrode array. LFPs are transient electrical signals generated in nerves and other tissues by the summed and synchronous electrical activity of the individual cells (e.g. neurons) in that tissue. LFP are "extracellular" signals, they are generated by transient imbalances in ion concentrations in the spaces outside the cells, that result from cellular electrical activity. LFP are 'local' because they are recorded by an electrode placed nearby the generating cells. Determining the local field potentials (LFPs) of the neuronal cells in the assembly can be performed with any suitable technology that is known to the person of skill and/or described herein. Examples are large-scale array recording simultaneously from 4,096 electrodes used to study propagating spontaneous and evoked network activity in acute murine cortico-hippocampal brain slices at unprecedented spatial and temporal resolution (see, for example, Hu X, Khanzada S, Klütsch D, Calegari F, Amin H. Implementation of biohybrid olfactory bulb on a high-density CMOS-chip to reveal large-scale spatiotemporal circuit information. Biosens Bioelectron. 2022 Feb 15;198:113834. doi: 10.1016/j.bios.2021.113834. Epub 2021 Nov 24. PMID: 34852985, and Emery BA, Hu X, Khanzada S, Kempermann G, Amin H. High-resolution CMOS-based biosensor for assessing hippocampal circuit dynamics in experience-dependent plasticity. Biosens Bioelectron. 2023 Oct 1;237:115471. doi: 10.1016/j.bios.2023.115471. Epub 2023 Jun 12. PMID: 37379793).

While the embodiment of MEA-seqX as described herein relates to the CMOS-MEA (4096 electrodes) (13,16) built upon active-pixel sensor technology (54), the method is not limited to a particular technology, it is also suitable to accommodate a spectrum of high-density technologies, such as those offered by switch-matrix technology (26,400 electrodes) (55), among others. MEA-seqX's application extends beyond *ex vivo* applications, encompassing expansive *in vivo* investigations.

Preferred is the method according to the present invention, wherein the recording of the patterns of local field potentials (LFPs) comprises the use of a high-density CMOS-based biosensing MEA chip (CMOS-MEA).

In the preferred embodiment of the method according to the present invention, the functional neuronal cell assembly that is characterized is a neuronal tissue (assembly) in the form of a dorsal horizontal brain slice of a thickness of about 300 $\mu$m. In the context of the present invention, any thickness of the neuronal tissue (consisting of cell assemblies) may be used for the LFP analysis that still remains functional and thus allows for the recording of suitable data. Examples are slices/tissue samples between about 500 $\mu$m and 100 $\mu$m, preferably between about 200 $\mu$m and 400 $\mu$m.

Preferred is a method according to the present invention, wherein the assessment and allocation of the recorded LFPs to specific features and shapes of waveforms from the interconnected slice/tissue and thus network layers comprises a grouping of the electrodes as used in the array and the use of principal component analysis (PCA) and K-means clustering algorithms.

The recording of the patterns of local field potentials (LFPs) is performed while obtaining spatial localization patterns of the LFPs of substantially the entire assembly using optical imaging. The imaging is used in order to provide a localization and/or allocation of the signals as detected with respect to the array as used. In view of the aim to provide precise spatial and temporal dynamics of the LFPs in order to provide suitable, standardized and reproducible data for further analysis, preferred is the method according to the present invention, wherein the **term "while" shall mean a time span that is less than 30 seconds, preferably less than 10 seconds,** more preferred less than 1 second, most preferred recording of the LFPs and the optical imaging is performed simultaneously.

**In the context of the present invention, the term "substantially" shall mean an area, surface or** number that allows to detect, determine and thus to calculate or predict information or data that is of statistically high relevance for the whole area, surface or number as analyzed. In one example according to the invention, substantially the entire assembly of cells is analyzed using optical imaging in order to obtain spatial localization patterns of the LFPs. Consequently, the area as analyzed must be sufficient to obtain spatial localization patterns of the LFPs with statistically high relevance for the whole assembly of cells as present in the preparation as used. Statistically high relevance is *at p < 0.05,* preferably at *p < 0.01,* more preferably *p < 0.001* (e.g. *ANOVA*).

Preferred is the method according to the present invention, wherein the optical imaging comprises imaging of the entire network of cell assemblies, for example using bright-filed imaging, comprising the use of a microscope, such as a modular stereomicroscope that may be equipped with a high-resolution camera.

Since the cells assemblies as analyzed need to be kept alive, in particular when studying the use of compounds that may influence the characteristics and/or activity of the cells (see below), preferred is a method according to the present invention, wherein the neuronal cell assembly is continuously perfused with a suitable perfusate solution during recordings. Suitable media are known to the person of skill and are described herein.

In a next step, subsequently, optical imaging and spatially resolved transcriptomic analysis of the cells in the assembly is performed using a second suitable array. This may be done directly following the above recording. Nevertheless, preferred is the method according to the present invention, wherein substantially the entire assembly is cryopreserved and may be stored before further transcriptome analysis. In order to preserve the state of the assembly that was the basis for the recording of LFPs, a rapid-freezing method is required. Preferably, the method of the present invention comprises cryopreserving comprising rapid freezing, preferably over dry ice, and optionally further comprises storing at a suitable temperature, for example about -80°C. Further preferred is the method according to the present invention, wherein the cell assemblies are suitably stained before the transcriptomic analysis, for example H&E as described herein. Respective stainings are known to the person of skill as well.

In order to further optimize the number of cells per spot and provide a clearer transcriptomic profile, the thickness of the neuronal assemblies may be further reduced before performing the transcriptome analysis. For this, the slices may be further horizontally cryosectioned to a thickness of between about 50 $\mu$m to 10 $\mu$m, preferably to between about 25 $\mu$m to 15 $\mu$m, most preferably to about 18 $\mu$m before transcriptome analysis.

As above, preferred is the method according to the present invention, wherein the optical imaging for the transcriptome analysis comprises imaging of the entire network of cell assemblies, for example using bright-filed imaging, comprising the use of a microscope, such as a modular stereomicroscope that may be equipped with a high-resolution camera.

Then, the spatially (locally) resolved transcriptomic analysis of the cells in the assembly is performed using a second suitable array. Methods for a spatially (locally) resolved transcriptomic analysis on arrays are known in the art (for example, in Duan H, Cheng T, Cheng H. Spatially resolved transcriptomics: advances and applications. Blood Sci. 2022 Nov 4;5(1):1-14. doi: 10.1097BS9.0000000000000141. PMID: 36742187; PMCID: PMC9891446; or Wang X, Almet AA, Nie Q. The promising application of cell-cell interaction analysis in cancer from single-cell and spatial transcriptomics. Semin Cancer Biol. 2023 Oct;95:42-51. doi: 10.1016/j.semcancer.2023.07.001. Epub 2023 Jul 15. PMID: 37454878; PMCID: PMC10627116), and as described herein.

Preferred is the method according to the present invention, wherein the transcriptomics comprises the generation of barcode-labeled cDNA and sequencing, such as quantitative sequencing, on the second array, such as, for example, spatially-resolved transcriptomics (SRT) sequencing.

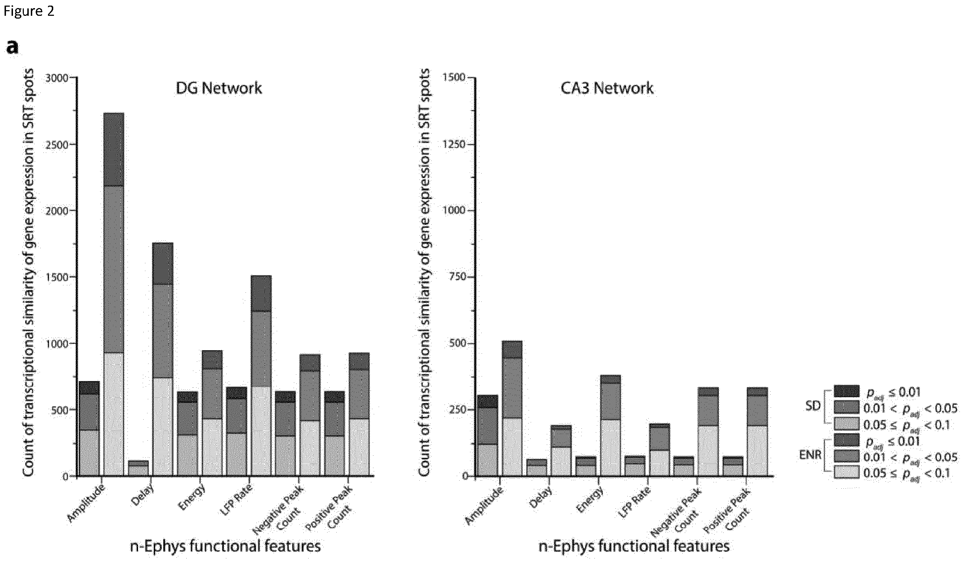

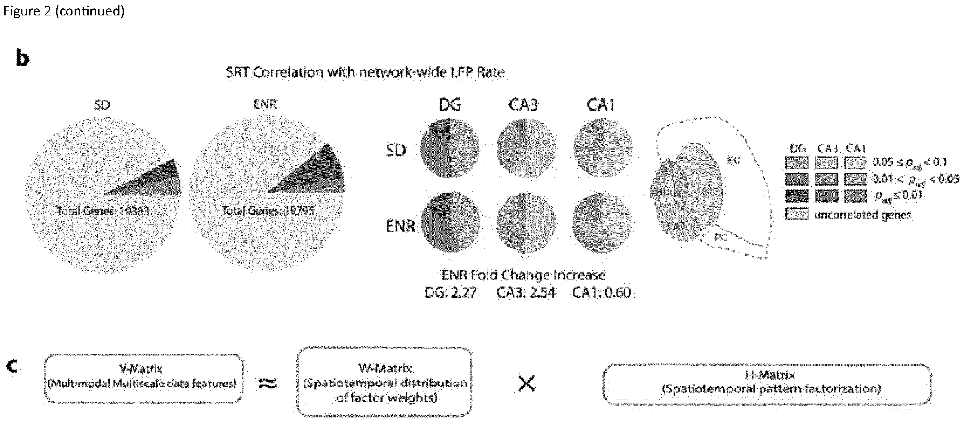

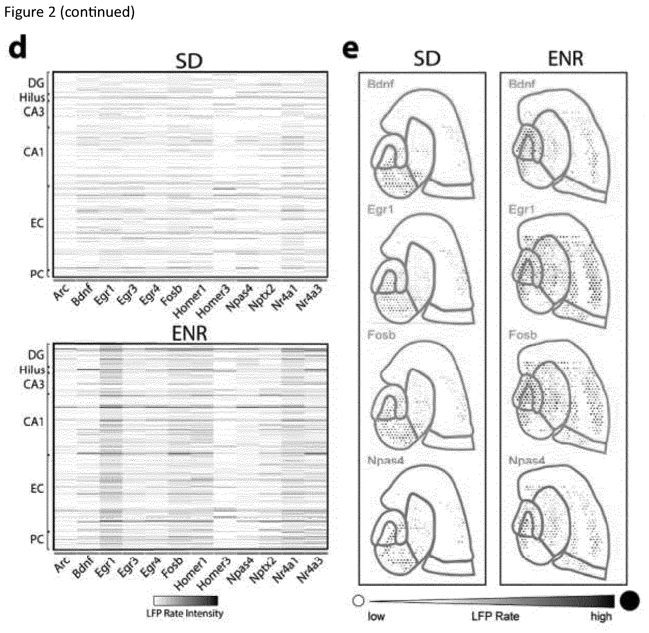

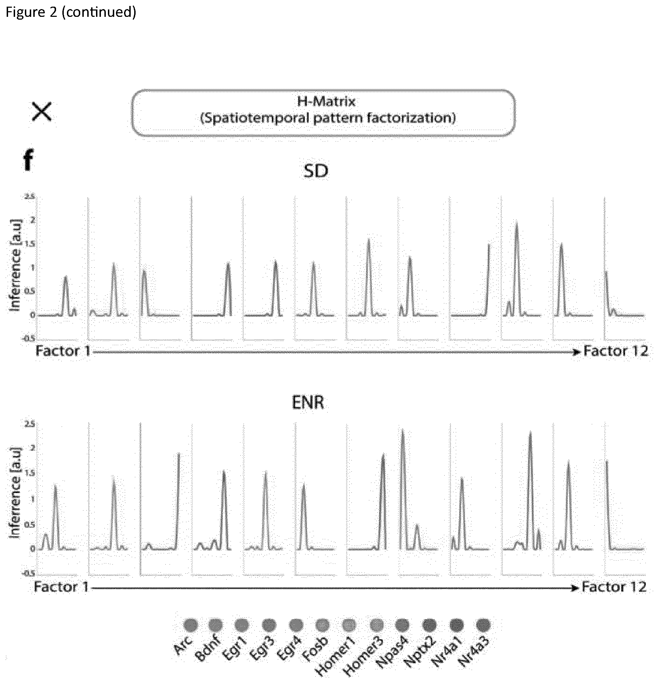

Another important aspect of the method according to the present invention relates to the collection, processing and use of the large amounts of data that are generated in the above steps. The data as obtained needs to be processed in order to align the spatial localization patterns of the LFPs with the spatially resolved sequencing and transcriptomic analysis data. Here, a combined input expression matrix (V) captures the collective information from SRT and n-Ephys (LFP rate) datasets (see Figure 2. Application of unsupervised machine learning, e.g. through sparsity-constrained NMF, enables the joint analysis of SRT and LFP (n-Ephys) data, revealing diverse subnetworks of genes, spatial locations, and electrophysiological features. Harmonizing data sources toward a common high-resolution spatiotemporal genomic-functional dimension and representation preferably uses a computational pipeline in Python.

As disclosed herein, one of the main goals of the method is to provide the timing and topology of the activity of the assembly in one high-resolution representation. Thereby, the spatiotemporal electrophysiological dynamics and spatial transcriptional profiles of cells in a functional neuronal cell assembly are detected. The inventive methods graph-theoretic analysis in the preferred embodiment as described herein revealed a small-world topology with densely connected hub complexes, indicating molecular-functional specialization and increased global communication capacity across scales (29,32,50). Furthermore, by combining DPT and CAT analyses, the method traced the pseudotemporal ordering along the developmental trajectory of cells within their spatial contexts and the progression of large-scale neural activity. This provides unique insights into coordinated spatiotemporal dynamics in the hippocampal circuitry.

Preferred aspects of the method according to the present invention are that processing the data and aligning the spatial localization patterns of the LFPs with the spatially resolved sequencing and transcriptomic analysis, comprises overlaying the optical images with the electrode array layout, and overlaying the optical images with the second array layout, and alignment of the two overlays comprising image resizing and rotation.

What sets the method according to the present invention apart is its unprecedented capability to simultaneously capture and integrate molecular and functional information across multiple spatiotemporal scales within intact brain tissues. While existing technologies have provided valuable insights, the method according to the present invention combines the advantages of various techniques, pushing the boundaries further. It presents a distinctive solution to the limitation of spatiotemporal resolution by harnessing the high-density capabilities of CMOS-MEA technology combined with spatial transcriptomics, optical imaging, and computational tools.

The method according to the present invention offers several advantages over the methods according to the state of the art:

- It allows for the generation of knowledge concerning the molecular structure and profiles of complex and extensive neuronal cell/brain activity;
- It provides a comprehensive view from the molecular to the functional network-wide levels, which was not possible with previous methods and thus only studied independently and individually.
- The method predictive capabilities using machine learning algorithms allow for an accurate prediction of network-wide electrophysiological features based on spatial gene expression, showing a multiscale causal relationship between specific gene expressions and neural activity;
- As mentioned herein, the method can be adapted beyond neural cell assemblies, offering additional applications in other electrogenic tissues like the olfactory bulb and cardiac tissues, providing insights into various biological contexts.

To illustrate the power of the inventive approach, the inventors applied it to the classical enriched environment paradigm of experience-dependent plasticity, in which the experimental intervention lies in differential housing conditions of laboratory mice. The enriched mice (ENR) lived in a larger group of isogenic animals in a larger enclosure compared to standard-housed animals (SD) (16,21). This highly influential paradigm elicits structural and functional changes throughout the brain and in the hippocampus. The inventors had recently been able to demonstrate that its effects on an unexpected scale relate to changes in the hippocampal circuit level (16).

According to the present invention, the embodiment MEA-seqX allows exploring how the computational dynamics and connectome of a large-scale hippocampal network are connected to the underlying transcriptional profile **dynamics. The inventor's method enables insight into** the causal link between these two dynamics. The invention allows identifying the molecular identity and dynamics of large-scale neural circuits, and thereby allows for the identification and development of new multimodal models and biomarkers of high functional validity in the contexts of health and disease. Such biomarkers are then suitable for the development of diagnostics and screening tools, especially in, but not limited to, precision medicine.

The method according to the present invention also allows cell-type identification in the neuronal cell assemblies and highlights the heterogeneity of neural cell types and their network-wide spectral fingerprints in the neuronal cell assemblies, here exemplified in the hippocampal circuit. Furthermore, the platform's predictive capabilities using machine learning algorithms allow for an accurate prediction and forecast of network-wide electrophysiological features based on spatial gene expression data, showing a multiscale causal relationship between specific gene expressions and neural activity, which offers a deeper understanding of neural dynamics. This also promotes research in machine learning and artificial intelligence.

The method according to the present invention in another aspect of the present invention provides for identifying the composition of functional neuronal assemblies. The method comprises performing the method according to the present invention as disclosed herein, and further comprising the step of concluding on the composition of the functional neuronal cell assemblies based on the spatiotemporal electrophysiological dynamics and transcriptional profiles of the cells as detected.

Further preferred is the method according to the present invention, wherein the identifying comprises landscaping or mapping of the cells in the assembly, the identification of clusters of cells and/or cell types, such as functional clusters, of the cells in the assembly, the identification of diverse neural cell types in the assembly, and/or the identification of electrical or spectral characteristics of cells in the assembly.

This allows the inventors to determine cell types and local tissue composition from the deconvolved gene expression patterns in order to construct multiscale spatial maps of the heterogeneity of neural types and their firing characteristics in the same neuronal, e.g. hippocampal-cortical tissue.

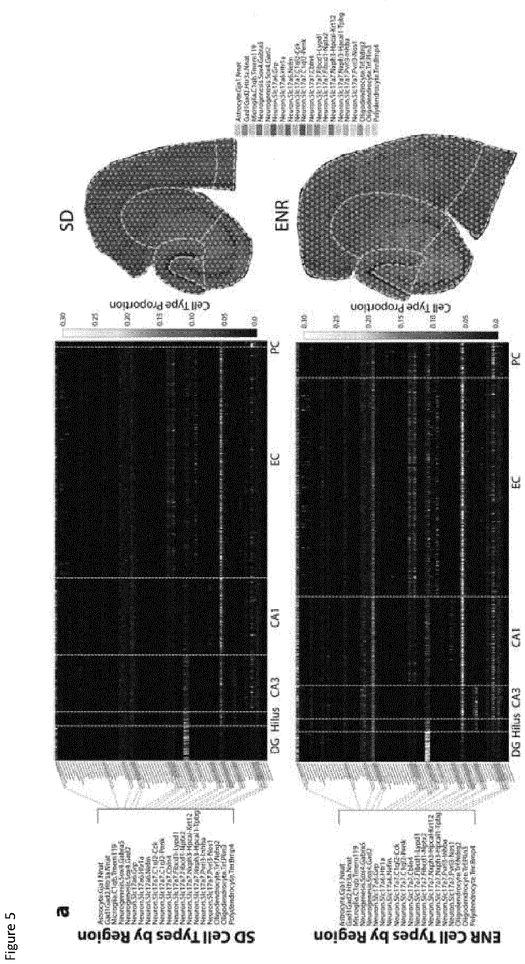

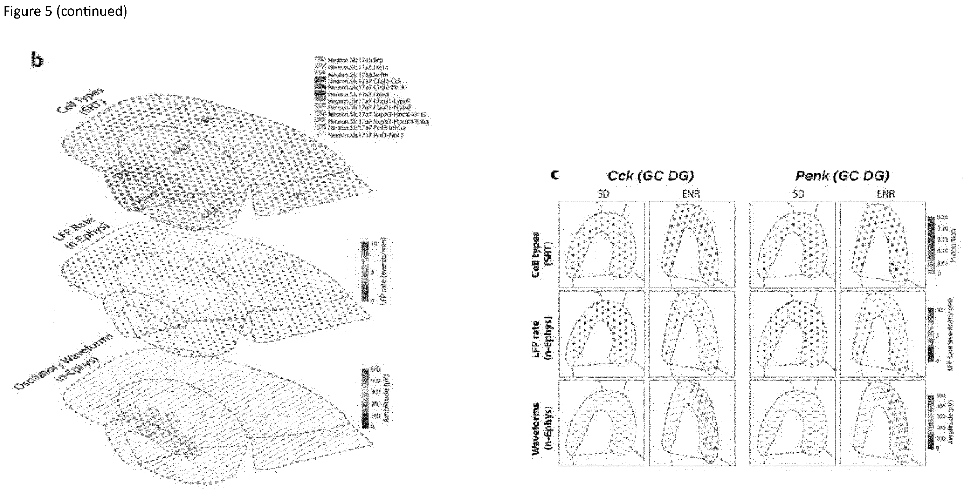

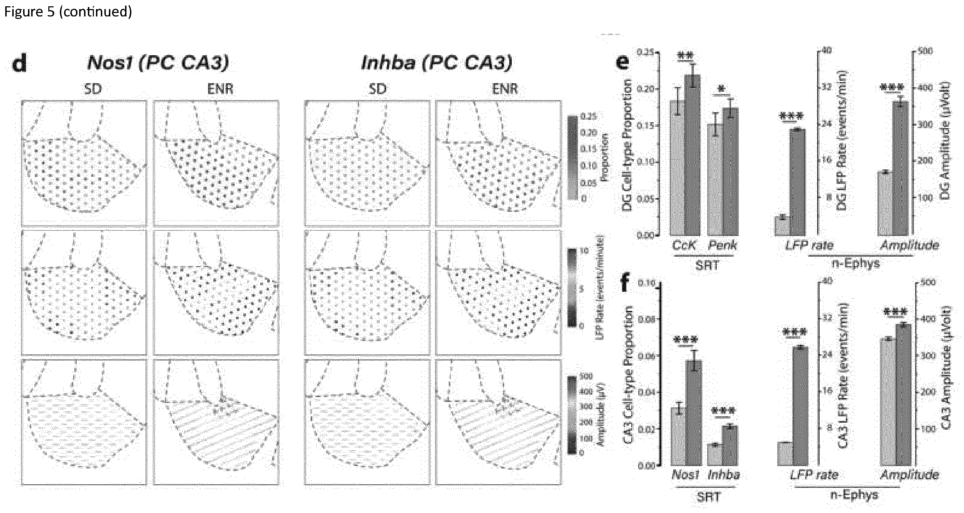

As an example, the analysis in the CA3 region revealed increased proportions of spatially localized marker genes *Nos1* and *Inhba*, linked to pyramidal cell types (see also Figure 5). The initial application of CARD yielded a broad group classification of hippocampal cell types. This diverse group encompassed astrocytes, endothelial cells, ependymal cells, macrophages, microglial cells, neurogenic cells, neurons, oligodendrocytes, and NG2 cells. Prior to any filtering, the inventors identified 85 different cell types, a finding that underscored the **experimental validity of the inventor's slice acquisition technique. Following the removal of** low-count cell types, the inventors were left with a robust group of 76 cell types. Interestingly, when these cell types were exposed to two different transcriptomic inputs from SD and ENR, the inventors observed a consistent distribution of prominent cell types across both **transcriptomes (Figure 5a). This result underscores the robustness of the inventor's approach** and the **reproducibility of the inventor's findings.**

Similar to the above, the method according to the present invention in another aspect of the present invention provides for monitoring the cellular composition of a functional neuronal cell assembly, comprising performing the method according to the present invention repeatedly at different time points, and monitoring the cellular composition of the functional neuronal cell assembly based on comparing the cellular compositions of the functional neuronal cell assembly as detected at different timepoints.

The method according to the present invention in another aspect of the present invention provides for monitoring the spatiotemporal electrophysiological dynamics and transcriptional profiles of functional neuronal cell assemblies comprising performing the method according to the present invention repeatedly at different time points, and monitoring the spatial electrophysiological and transcriptional information of the molecular neuronal cell assemblies based on comparing the spatiotemporal electrophysiological dynamics and transcriptional profiles of the cells as detected at different timepoints.

Preferred is the method according to the present invention, wherein the functional neuronal cell assembly to be monitored and/or otherwise characterized is selected from a neuronal tissue derived from a mammal having a behavioral condition or disorder, a mammal having a neurological condition or disorder, such as a neurodegenerative disorder or cancer, and/or a mammal being treated for one of these conditions or disorders.

In the above aspects, the present methods monitor, analyze, register, and/or detect changes in the functional neuronal cell assemblies over time. The repeating can be done once, twice, or more. Since the structure of the neuronal cell assemblies is destroyed when performing the transcriptomic analysis steps of the method according to the present invention, the repeated tests are performed with a functionally related sample, for example a neighboring brain slice, a second batch from the sample of neurological cancer, a second sample of the culture of interconnected brain cells, of the cultured neuronal differentiated iPSCs, a neighboring spinal marrow sample, and/or a neighboring sample of the electrogenic tissues like the olfactory bulb and cardiac tissues.

The monitoring may further include the analysis of selected biomarkers during the transcriptomic analysis steps of the method according to the present invention as described herein.

The monitoring according to the invention may also be performed in the context/as part of the identifying of a compound having an effect on the spatiotemporal electrophysiological and transcriptional profile and/or composition of cells in a functional neuronal cell assembly (see below), i.e. in the presence or absence of the compounds to be screened.

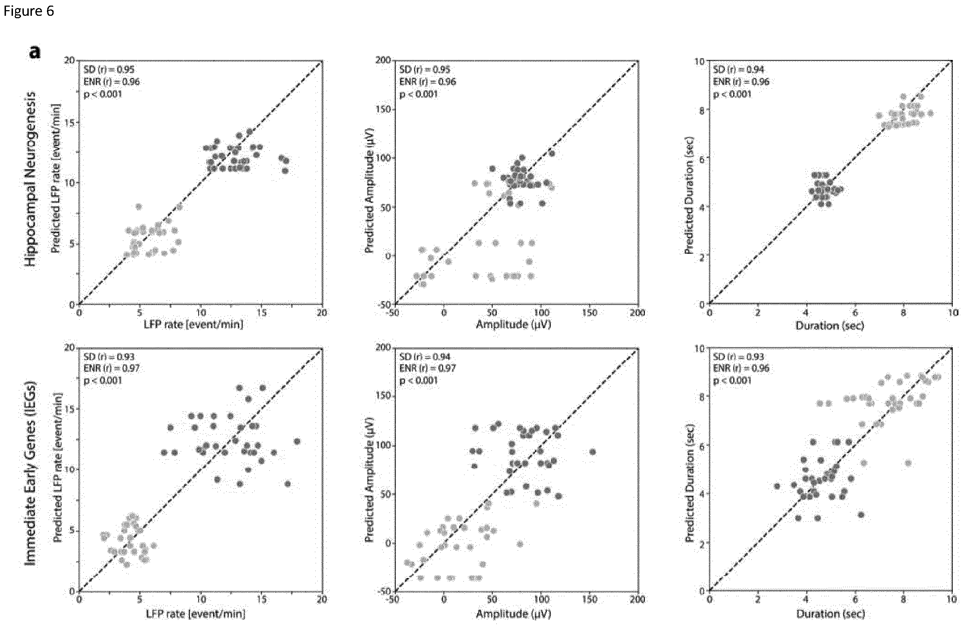

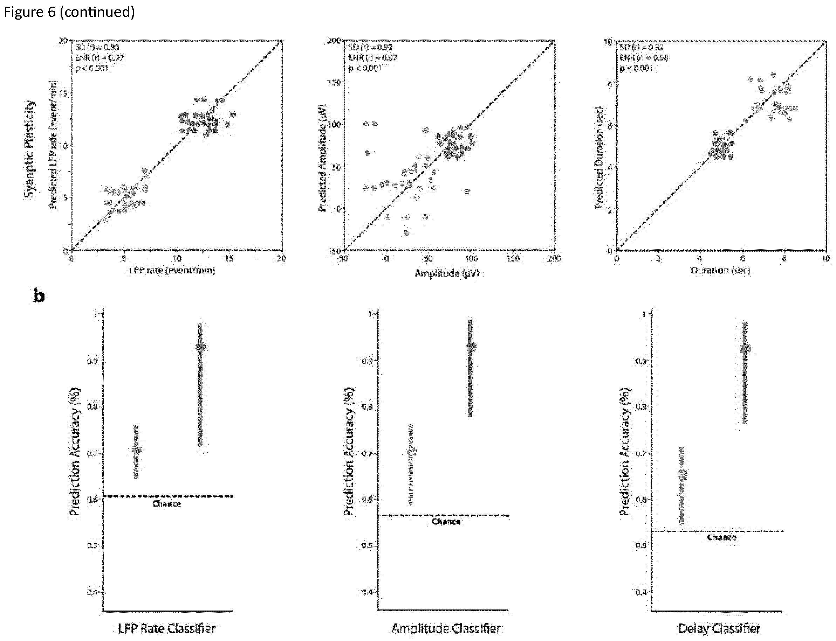

The method according to the present invention in another aspect of the present invention provides for predicting the spatiotemporal electrophysiological dynamics and/or behavior of functional neuronal cell assemblies. The method comprises performing the method according to the present invention, and further comprising providing spatially-resolved transcriptomic data of a neuronal cell assembly to be predicted. Then, a prediction of the (specific) electrophysiological dynamics and/or behavior of the assembly can be made, based on the data as provided as described herein (see also Figure 6).

As an example of how to investigate whether the expression profile of individual spatially-resolved genes can predict HC network-wide electrophysiological features, the inventors employed the Gradient Boosting (XGBoost) Algorithm, known for its strong interpretability by integrating multiple tree models (47). While previous methods have focused on gene properties correlated with electrophysiological and morphological diversity across cell types using low-resolution transcriptomics and electrophysiology (e.g., single-cell RNA sequencing and patch-clamp) (4,5,38), the method according to the present invention assesses whether specific electrophysiological features can be predicted using spatially-resolved transcriptomic data. The XGBoost model was trained for each quantitative n-Ephys measure using 70% of spatial transcriptomic data points in the detected SRT spots (i.e., 333 genes across the spatial context of HC tissue and six gene families as input). Three spatiotemporal n-Ephys metrics (LFP rate, amplitude, and time delay) were successfully predicted based on the differential spatial gene expression. The relationship between cross-validated predictions and the ground truth was evaluated with the Pearson correlation coefficient (r) for SRT-spot-n-Ephys electrodes (Figure 6a and Figure 12). By implementing the XGBoost on specific gene families that exhibited higher expression in ENR compared to SD circuits, the inventors observed significantly higher prediction accuracy for the ENR dataset. The XGBoost classifier achieved ~93% accuracy for the ENR dataset and ~70% for the SD dataset (Figure 6b). These results underscore a multiscale causative association between neural activity, plasticity, and distinct spatial gene expressions within specific gene families, and align with the predictions of network-level functionality modulated by prior experience (16). This approach according to the invention sheds light on critical genes and cellular pathways shaping neuronal responses and overall brain function while revealing the regulatory mechanisms governing neural dynamics, plasticity, and disease **pathogenesis. The inventor's integrated approach will massively reduce experimental** complexity. Specific genes predicted to influence particular electrophysiological features may then be targeted for manipulation to validate their functional role (48) and may be used for screening for agents that influence their role(s) in the prevention or treatment of diseases.

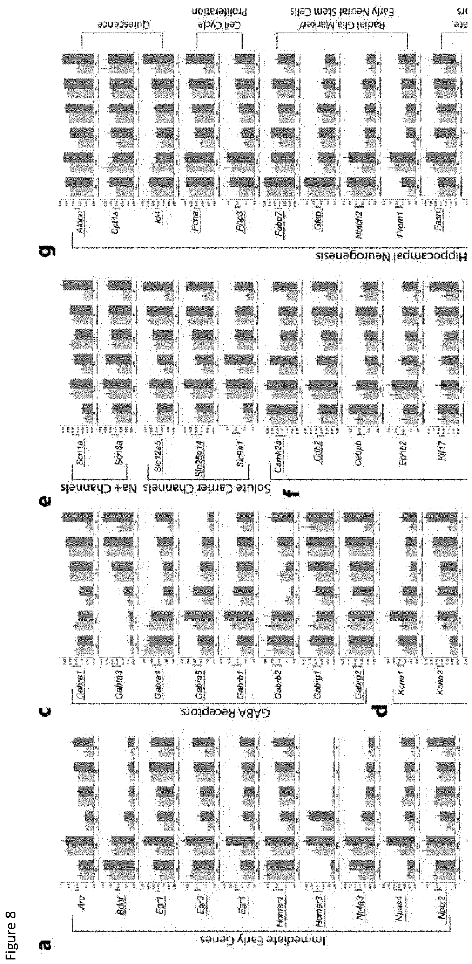

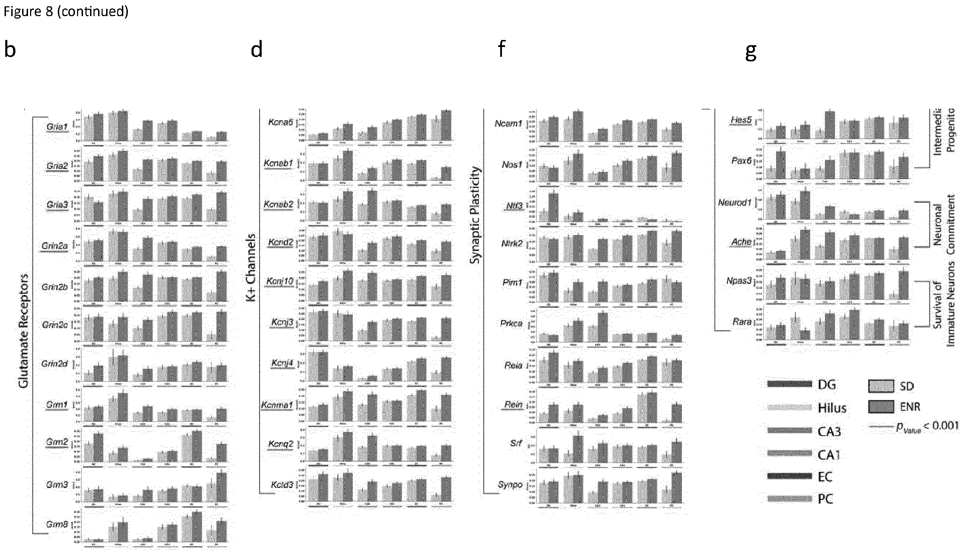

Notably, key genes critical for neuronal activity and function, such as *Bdnf*, *Egr1*, *Homer1*, *Npas4*, *Gria2*, and *Campk2a*, display heightened expression levels within the DG transcriptome of ENR, underscoring the enriched transcriptional landscape induced by environmental enrichment (Figure 8)

Yet another important aspect of the present invention then relates to a method for identifying a compound having an effect on the spatiotemporal electrophysiological and transcriptional profile and/or composition of cells in a functional neuronal cell assembly comprising performing the method according to the present invention in the presence and absence of at least one candidate compound, wherein a difference of the spatiotemporal electrophysiological dynamics and transcriptional profile and/or composition of cells in the functional neuronal cell assembly in the presence and absence of the at least one candidate compound or when compared to a suitable control identifies a compound having an effect on the spatiotemporal electrophysiological dynamics and transcriptional profile and/or composition of cells in a functional neuronal cell assembly.

Preferred is the method according to the present invention, wherein the effect is a therapeutic effect, such as a modification of the cellular composition, changes in gene expression changes in electrophysiological dynamics and properties, an anti-cancer effect, a neuroprotective effect, a morphological effect, a cognitive effect and/or a behavioral effect. Respective changes can be identified as described herein and/or include methods known to the person of skill. The understanding of the relationship between gene expression patterns and neural activity further allows the early detection and prevention of neurological disorders. For example the identifying of a specific gene expression profile linked to certain neural activities allows for the development of targeted interventions to modify or prevent these neural activities, thereby offering a preventive approach to neurological diseases.

In the context of the present invention, the candidate compound can be selected from the group consisting of a chemical organic molecule, a molecule selected from a library of small organic molecules (molecular weight less than about 500 Da), a molecule selected from a combinatory library, a cell extract, in particular a plant cell extract, a small molecular drug, a protein, a protein fragment, a molecule selected from a peptide library, an antibody or antigen binding fragment thereof, or a nucleic acid, a DNA, an RNA, or an siRNA.

These candidate molecules may also be used as a basis to screen for improved compounds. Thus, preferred is the method according to the present invention, wherein after the identification of the compound and/or a change/effect on the spatiotemporal electrophysiological dynamics and transcriptional profile and/or composition of cells in a functional neuronal cell assembly, the method further comprises the step of chemically modifying the compound. In general, many methods of how to modify compounds of the present invention are known to the person of skill and are disclosed in the literature.

Modifications of the compounds will usually fall into several categories, for example a) mutations/changes of amino acids into different amino acids, b) chemical modifications, e.g., through the addition of additional chemical groups, c) changes of the size/length of the compound, and/or d) the attachment of additional groups to the molecule (including marker groups, labels, linkers or carriers, such as chelators). In a next step, the modified compound is tested again in at least one of the tests as above, and if the property of the compound is improved **compared to its unaltered state. In the context of the present invention, an "improved" activity** relates to a desired increase or decrease of a change/effect on the spatiotemporal electrophysiological dynamics and transcriptional profile and/or composition of cells in a functional neuronal cell assembly.

Another aspect of the present invention relates to a method for producing a pharmaceutical composition, comprising performing a method for identifying a compound according to the present invention, and admixing the compound as identified with at least one pharmaceutically carrier.

The term "pharmaceutical composition" refers to a preparation which is in such form as to permit the biological activity of an active ingredient contained therein to be effective, and which contains no additional components which are unacceptably toxic to a subject to which the composition would be administered. A pharmaceutical composition of the present invention can be administered by a variety of methods known in the art. As will be appreciated by the skilled artisan, the route and/or mode of administration will vary depending upon the desired results. Pharmaceutically acceptable diluents include saline and aqueous buffer solutions. A "pharmaceutically acceptable carrier" refers to an ingredient in a pharmaceutical formulation, other than an active ingredient, which is nontoxic to a subject. Pharmaceutically acceptable carriers include any and all solvents, dispersion media, coatings, antibacterial and antifungal agents, isotonic and absorption delaying agents, and the like that are physiologically compatible. The carrier can be suitable for intravenous, intramuscular, subcutaneous, parenteral, spinal or epidermal administration (e.g., by injection or infusion).

The pharmaceutical compositions produced according to the invention may also contain adjuvants such as preservatives, wetting agents, emulsifying agents and dispersing agents. Prevention of the presence of microorganisms may be ensured both by sterilization procedures and by the inclusion of various antibacterial and antifungal agents, for example, paraben, chlorobutanol, phenol, sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin. The pharmaceutical compositions produced according to the invention may be in liquid, dry or semisolid form, such as, for example, in the form of a tablet, coated tablet, effervescent tablet, capsule, powder, granulate, sugar-coated tablet, lozenge, pill, ampoule, drop, suppository, emulsion, ointment, gel, tincture, paste, cream, moist compress, gargling solution, plant juice, nasal agent, inhalation mixture, aerosol, mouthwash, mouth spray, nose spray, or room spray. Preferred is an injectable composition.

Another aspect of the present invention relates to a device that is specifically suitable for performing the method according to the present invention as described herein, in particular a device comprising the neuronal cell assembly to be analyzed, an electrode array suitable to record patterns of local field potentials (LFPs) of the assembly, while performing optical imaging, a second array suitable to perform spatially resolved transcriptomic analysis of the cells in the assembly while also performing optical imaging, and a computer program product for processing the data as obtained comprising programming means for collecting and processing data as generated, in particular for aligning the spatial localization patterns of the LFPs with the spatially resolved sequencing and transcriptomic analysis and/or principal component analysis (PCA) and K-means clustering algorithms, optionally with additional buffers (see, for example, examples, below), and instructions for use.

Another aspect of the present invention relates to the use of the device according to the present invention for detecting the spatiotemporal electrophysiological dynamics and transcriptional profiles of cells in a functional neuronal cell assembly, for identifying the composition of a functional neuronal cell assembly, for monitoring the spatial electrophysiological dynamics and transcriptional profiles of cells in a functional neuronal cell assembly, for monitoring the cellular composition of a functional neuronal cell assembly, or for identifying a compound having an effect on the spatiotemporal electrophysiological dynamics and transcriptional profiles and/or for identifying the composition of cells in a functional neuronal cell assembly.

Another aspect of the present invention then relates to a method for preventing or treating a neurological disease or disorder and/or improving cognitive function in a subject in need of said prevention, treatment or improvement, comprising administering to the subject an effective amount of the pharmaceutical compositions produced according to the invention.

Preferred is the method according to the present invention, wherein the neurological disease or disorder in the subject is selected from the group consisting of a disease or disorder causing an undesired change/effect on the spatiotemporal electrophysiological dynamics and transcriptional profile and/or composition of cells in a functional neuronal cell assembly, arrythmia, hormonal disorders, Morbus Parkinson, Morbus Alzheimer, dementia, behavioral disorders, cognitive disorders, brain cancer, stroke, depression, affective disorders, comorbid depressive symptoms, substance addiction and abuse, obsessive-compulsive disorders, personality disorders (e.g., borderline), autism, Tourette syndrome, and anxiety disorders.

**In the context of the present invention, a "subject" relates to a mammal, animal or individual,** such as a person or patient that undergoes prevention or treatment according to the invention against a psychiatric disorder or disease. In the context of the present invention, the term **"treatment" or "therapy" shall mean** the attempted remediation of a health problem as disclosed herein, i.e., the psychiatric disorder or disease in the subject.

**In the context of the present invention, the term "about" shall mean to include a deviation of** +/- 10% of the value as given, if not indicated otherwise.

The method according to the present invention introduces a platform that provides the unprecedented capability to simultaneously capture and integrate molecular and functional information across multiple spatiotemporal scales within intact neurological, such as brain, tissues. The method according to the present invention presents a distinctive solution to limited spatiotemporal resolution by harnessing the high-density capabilities of CMOS-MEA technology combined with spatial transcriptomics, optical imaging, and computational tools, offering exceptional temporal resolution down to individual cells in their spatial contexts. The present invention fills a crucial gap in the possibilities to understand the molecular infrastructure supporting and resolving the integrity of large-scale neuronal interactions in physiological and experience-dependent plasticity paradigms (18,49). Through the present integrative approach, the inventors identified spatially resolved causal regulation across molecular-functional features, which uncovered the impact of environmental factors on coordinated neural activity and gene expression - a suspected but previously essentially inaccessible link.

The platform's graph-theoretic analysis revealed a small-world topology with densely connected hub complexes, indicating molecular-functional specialization and increased global communication capacity across scales (29,32,50). Furthermore, by combining DPT and CAT analyses, the method according to the present invention traced the pseudotemporal ordering along the developmental trajectory of cells within their spatial contexts and the progression of large-scale neural activity, providing unique insights into coordinated spatiotemporal dynamics in the hippocampal circuitry. The method according to the present invention also demonstrated the potential for cell-type identification and highlighted the heterogeneity of neural cell types and their network-wide spectral fingerprints in the hippocampal circuit. The platform's predictive capabilities using machine learning algorithms allowed accurate forecasting of network-wide electrophysiological features based on spatial gene expression, showing a multiscale causal relationship between specific gene expressions and neural activity to offer a deeper understanding of neural dynamics, which opens new avenues for research in machine learning and artificial intelligence. Combining the method according to the present invention with intelligent neural networks enhances the understanding of complex data, decision-making processes, and learning mechanisms (51,52).

Furthermore, the potential of the method according to the present invention in personalized medicine is promising. The identification of spatiotemporal transcriptomic-electrophysiological biomarkers may aid in the early diagnosis and treatment of neurological disorders, ultimately leading to precision personalized therapies (53).

Moreover, in the context of other neurological and psychiatric disorders the method according to the present invention will deepen the understanding of disease mechanisms and therapeutic targets. The platform's potential to uncover molecular-functional changes associated with various disease states paves the way for developing novel treatments.

Further integrating additional modalities into the method according to the present invention, such as proteomic and epigenomic data, will provide even more comprehensive insights into neural information processing (51). The platform's adaptability goes beyond neural tissues, extending to electrogenic tissues like the olfactory bulb and cardiac tissues. This enables studying gene expression and electrophysiology interactions in diverse contexts. Its application in cardiac tissue offers insights into heart function, aiding cardiology advancements.

The method according to the present invention is further adapted to accommodate a spectrum of high-density technologies, such as those offered by switch-matrix technology (26,400 electrodes) (55), among others. Importantly, the use of the method according to the present invention extends beyond *ex vivo* applications, encompassing *in vivo* investigations. Integration with state-of-the-art *in vivo* probes, such as Neuropixels (56), SiNAPS (57), or other emerging modalities, offers the potential to study functional neural dynamics and gene expression patterns within living organisms, bridging the gap between laboratory findings and real-world biological contexts.

The platform according to the present invention, with its capability to simultaneously capture and integrate molecular and functional information across multiple spatiotemporal scales within intact brain tissues, has vast potential applications in both research and clinical settings.

Its primary application lies in providing a deeper understanding of the brain's computational principles across scales, bridging the gap between molecular architecture and brain activity. This can be pivotal in:

- Neurological Research: Understanding neural plasticity, connectivity dynamics, and molecular-functional shifts related to aging, development, and various neurological conditions.
- Drug Development and Testing: The platform can be used to test the effects of new drugs or treatments on neural activity and transcriptomic level simultaneously, providing insights into their efficacy and potential side effects.
- Diagnostic Tools: With its predictive capabilities using machine learning algorithms, MEA-seqX can be developed into diagnostic tools that can forecast network-wide electrophysiological features based on spatial gene expression.
- Precision Personalized Medicine: The platform holds potential for diagnostic tools and therapies tailored based on gene-neural electrophysiology features of individuals.
- Early Detection of Neurological Disorders (Prevention): The ability of MEA-seqX to detect subtle changes in gene expression and neural activity could facilitate the early diagnosis of neurological disorders. For example, certain gene expression patterns may be indicative of the early stages of neurodegenerative diseases like Alzheimer's or Parkinson's diseases, long before clinical symptoms manifest.
- Disease Progression Monitoring: The method's capability to track changes in neural activity and gene expression over time makes it an excellent tool for monitoring the progression of neurological disorders. This can help in adjusting treatment plans as the disease evolves.
- Biomarker Discovery: MEA-seqX's comprehensive data analysis can aid in the discovery of new biomarkers for neurological diseases. By identifying novel genes or neural activity patterns associated with specific conditions, it can broaden the scope of diagnostic markers available.
- Understanding Disease Mechanisms: The integration of genetic and neural data can provide insights into the mechanisms underlying neurological disorders. This deeper understanding can not only aid in diagnosis but also in developing more effective treatments.

In the context of the present invention, the methods as provided move beyond single-cell methods and focus on multiscale network-level dynamics. The present invention provides a more comprehensive and nuanced understanding of hippocampal-cortical cell types and their interactive sequential electrophysiological properties across multiple scales.

The present invention relates to the following items:

Item 1. An *ex vivo* method for detecting the spatiotemporal electrophysiological dynamics and transcriptional profiles of functional neuronal cell assemblies comprising providing a functional neuronal cell assembly to be characterized, recording patterns of local field potentials (LFPs) of the neuronal cells in the assembly at high spatial and temporal resolution using a suitable electrode array, and obtaining spatial localization patterns of the LFPs of substantially the entire assembly using optical imaging, optionally, cryopreserving substantially the entire assembly, performing optical imaging and spatially resolved transcriptomic analysis of the cells in the assembly using a second array, and processing the data as obtained comprising aligning the spatial localization patterns of the LFPs with the spatially resolved transcriptomic analysis, and thereby detecting the spatiotemporal electrophysiological dynamics and spatial transcriptional profiles of cells in the functional neuronal cell assembly.

Item 2. The method according to Item 1, wherein the functional neuronal cell assembly to be characterized is selected from a neuronal tissue, a brain slice, a sample of a neurological cancer, a culture of interconnected brain cells, cultured neuronal differentiated iPSCs, spinal marrow, electrogenic tissues like the olfactory bulb and cardiac tissues, and parts or combinations thereof, in particular a neuronal tissue derived from a mammal having a behavioral condition or disorder, a mammal having a neurological condition or disorder, such as a neurodegenerative disorder or cancer, and/or a mammal being treated for one of these conditions or disorders.

Item 3. The method according to Item 1 or 2, wherein recording of the patterns of local field potentials (LFPs) comprises the use of a high-density CMOS-based biosensing MEA chip (CMOS-MEA).

Item 4. The method according to any one of Items 1 to 3, wherein the optical imaging comprises imaging of the entire network of cell assemblies, for example using bright-filed imaging, comprising the use of a microscope, such as a modular stereomicroscope.

Item 5. The method according to any one of Items 1 to 4, wherein the neuronal cell assembly is continuously perfused with a suitable perfusate solution during recordings.

Item 6. The method according to any one of Items 1 to 5, wherein the recording of the LFPs and the optical imaging is performed simultaneously.

Item 7. The method according to any one of Items 1 to 6, wherein cryopreserving comprises rapid freezing, preferably over dry ice, and optionally further comprises storing at -80°C.

Item 8. The method according to any one of Items 1 to 7, wherein the cell assemblies are suitably stained before the transcriptomic analysis.

Item 9. The method according to any one of Items 1 to 8, wherein the transcriptomics comprises the generation of barcode-labeled cDNA and sequencing, such as quantitative sequencing, on the second array, such as, for example, spatially-resolved transcriptomics (SRT) sequencing.

Item 10. The method according to any one of Items 1 to 9, wherein the assessment and allocation of the recorded LFPs to specific features and shapes of waveforms from interconnected network layers comprises a grouping of the electrodes and the use of principal component analysis (PCA) and K-means clustering algorithms.

Item 11. The method according to any one of Items 1 to 10, wherein processing the data and aligning the spatial localization patterns of the LFPs with the spatially resolved sequencing and transcriptomic analysis, comprises overlaying the optical images with the electrode array layout, and overlaying the optical images with the second array layout, and alignment of the two overlays comprising image resizing and rotation.

Item 12. A method for identifying the composition of functional neuronal assemblies, comprising performing the method according to any one of Items 1 to 11, and further comprising the step of concluding on the composition of the functional neuronal assemblies based on the spatiotemporal electrophysiological dynamics and transcriptional profiles of the cells as detected.

Item 13. The method according to Item 12, wherein the identifying comprises mapping of the cells in the assembly, the identification of clusters, such as functional clusters, of the cells in the assembly, the identification of diverse neural cell types in the assembly, and/or the identification of spectral characteristics of cells in the assembly.

Item 14. A method for monitoring the spatiotemporal electrophysiological dynamics and transcriptional profiles of functional neuronal cell assemblies comprising performing the method according to any one of Items 1 to 11 repeatedly at different time points, and monitoring the spatial electrophysiological and transcriptional information of the molecular neuronal cell assemblies based on comparing the spatiotemporal electrophysiological dynamics and transcriptional profiles of the cells as detected at different timepoints.

Item 15. A method for monitoring the cellular composition of a functional neuronal cell assembly, comprising performing the method according to Item 12 or 13 repeatedly at different time points, and monitoring the cellular composition of the functional neuronal cell assembly based on comparing the cellular compositions of the functional neuronal cell assembly as detected at different time points.

Item 16. The method according to Item 14 or 15, wherein the functional neuronal cell assembly to be characterized is selected from a neuronal tissue derived from a mammal having a behavioral condition or disorder, a mammal having a neurological condition or disorder, such as a neurodegenerative disorder or cancer, and/or a mammal being treated for one of these conditions or disorders.

Item 17. A method for identifying a compound having an effect on the spatiotemporal electrophysiological and transcriptional profile and/or composition of cells in a functional neuronal cell assembly comprising performing the method according to any one of Items 1 to 13 in the presence and absence of at least one candidate compound, wherein a difference of the spatiotemporal electrophysiological and transcriptional dynamics and/or composition of cells in the functional neuronal cell assembly in the presence and absence of the at least one candidate compound or when compared to a suitable control identifies a compound having an effect on the spatiotemporal electrophysiological dynamics and transcriptional profile and/or composition of cells in a functional neuronal cell assembly.

Item 18. The method according to Item 17, wherein the effect is a therapeutic effect, such as a modification of the cellular composition, changes in gene expression, an anti-cancer effect, a neuroprotective effect, a morphological effect, a cognitive effect and/or a behavioral effect.

Item 19. A device for performing the method according to any one of Items 1 to 18, in particular comprising the neuronal cell assembly to be analyzed, an electrode array suitable to record patterns of local field potentials (LFPs) of the assembly while performing optical imaging, a second array suitable to perform spatially resolved transcriptomic analysis of the cells in the assembly while performing optical imaging, and a computer program product for processing the data as obtained comprising aligning the spatial localization patterns of the LFPs with the spatially resolved sequencing and transcriptomic analysis, optionally with additional buffers, and instructions for use.

Item 20. Use of the device according to Item 19 for detecting the spatiotemporal electrophysiological dynamics and transcriptional profile of cells in a functional neuronal cell assembly, for identifying the composition of a functional neuronal cell assembly, for monitoring the spatial electrophysiological and transcriptional dynamics of cells in a functional neuronal cell assembly, for monitoring the cellular composition of a functional neuronal cell assembly, or for identifying a compound having an effect on the spatiotemporal electrophysiological dynamics and transcriptional profile and/or for identifying the composition of cells in a functional neuronal cell assembly.

The invention will now be described further in the following examples with reference to the accompanying figures, nevertheless, without being limited thereto. For the purposes of the present invention, all references as cited are incorporated by reference in their entireties.
In the accompanying figures,

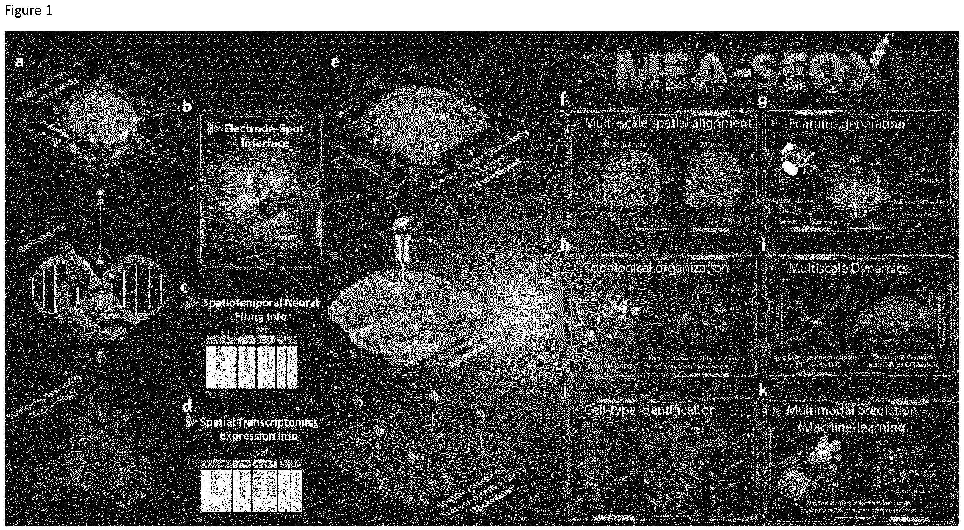

Figure 1 shows an MEA-seqX platform overview for integrating brain dynamics across scales. a), e) The platform combines high-density CMOS-MEA, optical microscopy, and spatial sequencing technology to examine the relationship between spatial transcriptomics and neural oscillatory dynamics, b) A closer examination, exemplified by a sensing electrode coupled with the SRT spot, highlights the discernible pitch size difference between the two platforms, c) A high-density CMOS-MEA equipped with 4,096 sensing electrodes adeptly captures functional firing LFP patterns emanating from all HC regions with high precision in spatial coordinates, d) Leveraging spatial barcodes, SRT with 5,000 spots allows the systematic mapping of gene expression profiles, elucidating the spatial nuances across HC regions, f) A Python-based pipeline processes data to map transcriptional-functional dynamics. This includes a multifaceted spatial rescaling and alignment approach, establishing a direct association between neural activity and transcriptomic features, g) Advanced analysis from multidimensional readouts provides insights into multiscale network features, h) Topological graphs capture the intricate web of multimodal transcriptional-functional connectivity, i) Multiscale neural dynamics are quantified with gene pseudotime and center of activity trajectories, j) Cell-type composition is inferred from spatial transcriptomic data correlated with their firing characteristics obtained from n-Ephys. k) Automatic machine-learning algorithm accurately predicts electrophysiological features from transcriptomic profiles.

Figure 2 shows the integration of spatial gene expression patterns and network electrophysiological features, a) Comparative analysis of SRT expression patterns and functional n-Ephys features illustrate enhanced gene expression patterns in ENR corresponding to the DG and CA3 regions compared to SD. The statistical significance of SRT and n-Ephys features correlation is quantified using Benjamini-Hochberg false-discovery rate (FDR)-adjusted *p-values* (*padj*). b) Quantitative profiling of correlated genes based on network-wide LFP rate reveals a 2.2-fold increase in correlated genes in the DG and 2.5-fold and 0.6-fold increases in CA3 and CA1 subregions, respectively, in ENR compared to SD. The significance of SRT and LFP correlation is measured using Benjamini-Hochberg FDR-adjusted p-values (padj). c) Application of unsupervised machine learning through sparsity-constrained NMF. The algorithm enables the joint analysis of SRT and n-Ephys data, revealing diverse subnetworks of genes, spatial locations, and electrophysiological features, d) The combined input expression matrix (V) captures the collective information from SRT and n-Ephys (LFP rate) datasets. Each entry signifies the gene expression level correlated to network function features at a specific spatial location, e) Visualization of spatial gene expression patterns through the basis matrix (W). This matrix illustrates how gene expression patterns are distributed across spatial locations and linked to functional features, f) Coefficient matrix (H) representing the contribution of basis vectors to spatial locations and n-Ephys features. The analysis reveals increased normalized gene expression and network feature values in the ENR network, emphasizing spatially resolved components driving gene intensity.

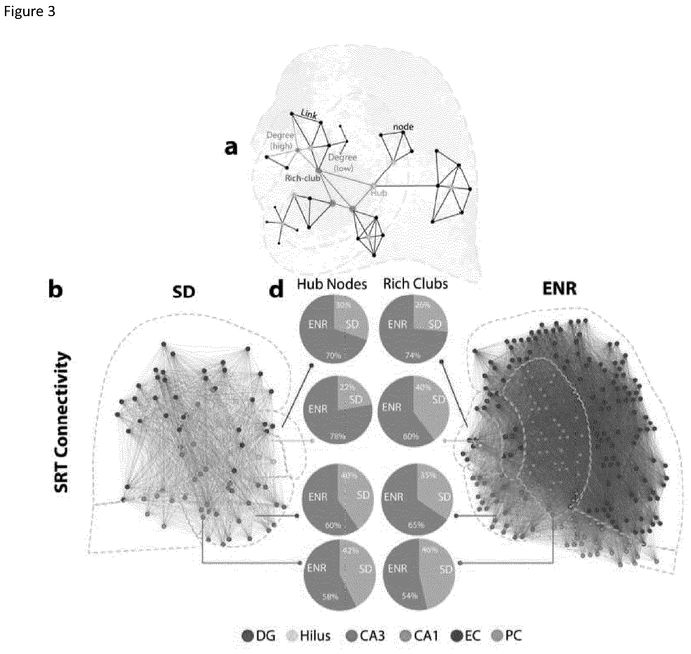

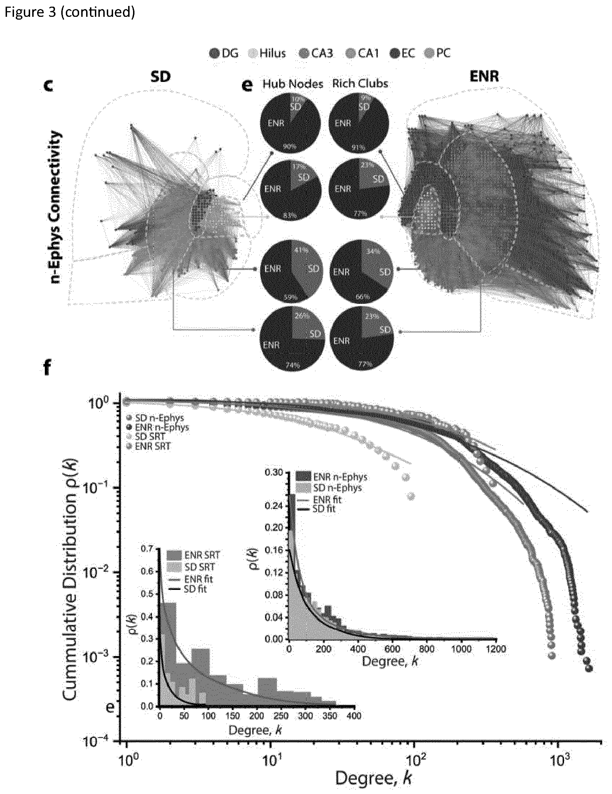

Figure 3 shows the multiscale network topological analysis of spatially sequenced connectome and neural functional connectivity, a) Illustration of key multiscale graph measures depict the characteristic Hub and Rich-club nodes defined based on their degree of interconnectedness in an acute hippocampal-cortical slice. Node degree corresponds to the number of attached links at a given node, b) Connectivity maps of spatial IEGs in HC-interconnected layers in ENR and SD. The networks are visualized with Gephi to illustrate total connections from complete sequenced SD data (node = 546, and links = 5,166) and ENR data (node = 877, and links = 50,974). c) Connectivity maps of spatiotemporal functional neural activity in HC-interconnected layers under ENR and SD conditions. The networks are visualized with Gephi to illustrate 2% of total connection in SD (node = 1,057, and links = 24,217) and ENR large-scale recorded data (node = 2,003, and links = 36,312). Graph nodes in (b) and (c) are scaled according to degree strength and colored according to HC module association and indicated in colored circles legends. Colored links identify the intra- and inter-cluster connections, d) The percent of quantified hubs and rich-club nodes in different hippocampal transcriptomic networks in SD and ENR. e) The percent of quantified hubs and rich-club nodes in different hippocampal functional networks in SD and ENR. f) The power-law distributions indicate scale-free transcriptomic (SRT)-functional (n-Ephys) network topologies with small-world properties in SD and ENR networks. The log-log plot of the cumulative connection distribution for ENR (SRT and n-Ephys; blue) networks exhibits a significantly heavier tail than SD networks (SRT and n-Ephys; grey), indicating low-degree nodes coexist with a few densely connected hubs, yet higher than SD networks, which reach a cut- Kolmogorov-Smirnov test). This is also supported at the linear scale (insets) for all conditions, and their compliance to power-law is assessed with Pareto fits (*$p < 0.05$ Kolmogorov-Smirnov test*). The lognormal function fitted the power-law distributions with goodness-of-fit in a log-log plot (with a coefficient of determination $R^2 = 0.95, 0.98, 0.96$, and $0.97$ for SRT, n-Ephys (SD), SRT, n-Ephys (ENR), respectively). The probability density function of Pareto fitted the power-law distributions with goodness-of-fit in the linear plots (with $R^2 = 0.96, 0.97, 0.95$, and $0.97$ for SRT, n-Ephys (SD), SRT, n-Ephys (ENR), respectively).

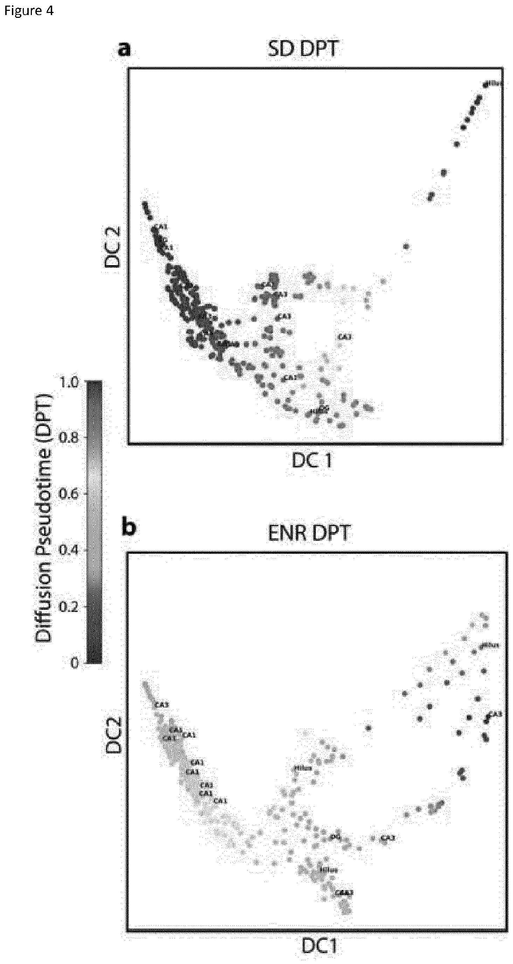

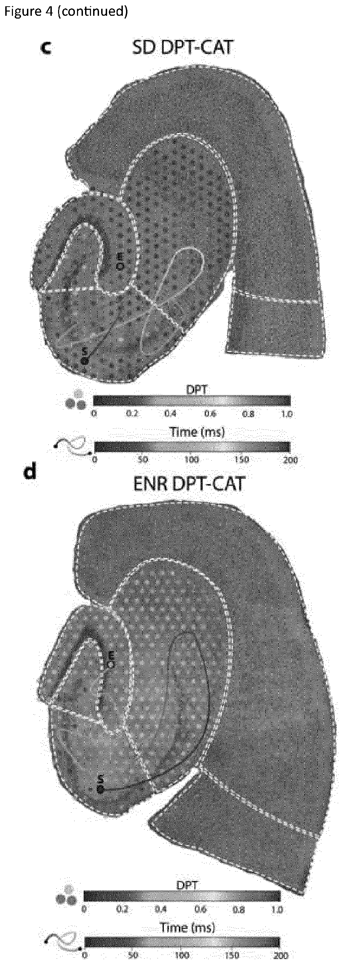

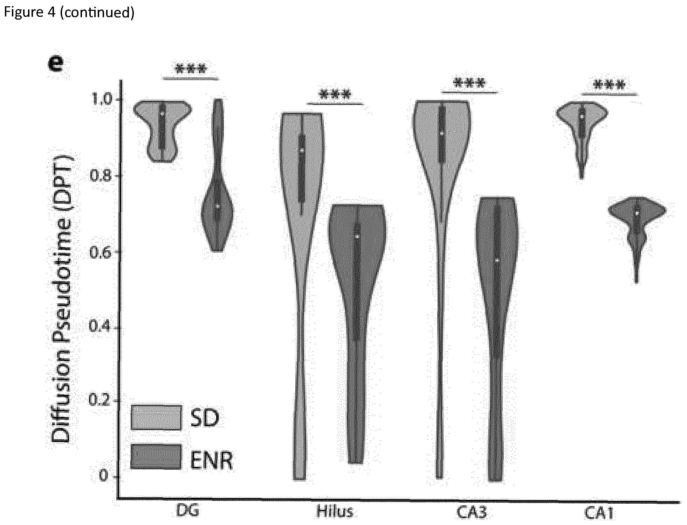

Figure 4 shows the analysis of multiscale hippocampal dynamics, a) Differential progression of DPT in the expression of IEGs from SRT SD data showcases the developmental trajectory of cells within the hippocampal spatial regions, b) Same as in (a) but in ENR. c) Correspondence between DPT and CAT analyses in SD to infer colocalization, spatial-temporal alignment, and functional insights, d) Same as in (c) but in ENR. e) Quantifying differential DPT progression in interconnected hippocampal regions in SD and ENR (*$p < 0.001$ ANOVA test*).

Figure 5 shows the analysis of spatial cell-type and their firing pattern fingerprints, a) A regionally ordered heatmap based on the CARD method depicts the cell type proportions of the 76 filtered cell types. Inset lists and localized cell types superimposed on SD and ENR brain slices highlight the 20 cell types with the highest proportions. Comparative analysis of hippocampal transcriptomes between SD and ENR conditions reveals a comparable distribution of prominent cell types, b) Integrating the CARD methodology into the MEA-seqX platform yields a spatially resolved composition of cell types accompanied by underlying oscillatory firing characteristics. This presentation includes the top 20 cell types with the highest proportions, as determined by SRT readouts, LFP Rate, and oscillatory waveforms derived from n-Ephys readouts across the entire hippocampal network, c) Examination focusing on the DG region offers an in-depth view of the spatially resolved composition of cell types alongside the corresponding oscillatory firing features, d) Similarly, a regional assessment within the CA3 pyramidal cell (PC) network provides insight into the spatial composition of cell types and their corresponding oscillatory firing features, e) The ENR transcriptome exhibits higher proportions of spatially localized marker genes - *Cck* and *Penk*-associated with granule cell types in the DG. These proportions are notably elevated compared to the SD transcriptome. Similarly, ENR exhibits a higher LFP rate and amplitude than SD (***$p < 0.001$, **$p < 0.01$, ANOVA, **$p < 0.05$, ANOVA). f) Analysis in the CA3 region reveals increased proportions of spatially localized marker genes *Nos1* and *Inhba*, linked to pyramidal cell types, in the ENR transcriptome compared to the SD transcriptome, which correlates with the higher LFP rate and amplitude on the functional network scale (***$p < 0.001$, ANOVA).

Figure 6 shows the machine-learning prediction for multiscale transcriptional-functional data, a) Application of the XGBoost algorithm to predict network electrophysiological metrics (LFP rate, amplitude, and duration) from transcriptomic data of each specified gene family in SD and ENR data. The prediction of n-Ephys metrics from the transcriptomics data is evaluated with Pearson correlation coefficient ($r$) in SD and ENR. The significant difference between the predicted SD and ENR values in all gene families is indicated (***$p < 0.001$, ANOVA). b) Performance of the XGBoost indicated by mean accuracy value comparison from final data output iterations of all gene families exhibiting higher prediction accuracy for the ENR than SD data. The values are computed over the mean, and three standard deviations are determined to be within the threshold of chance.

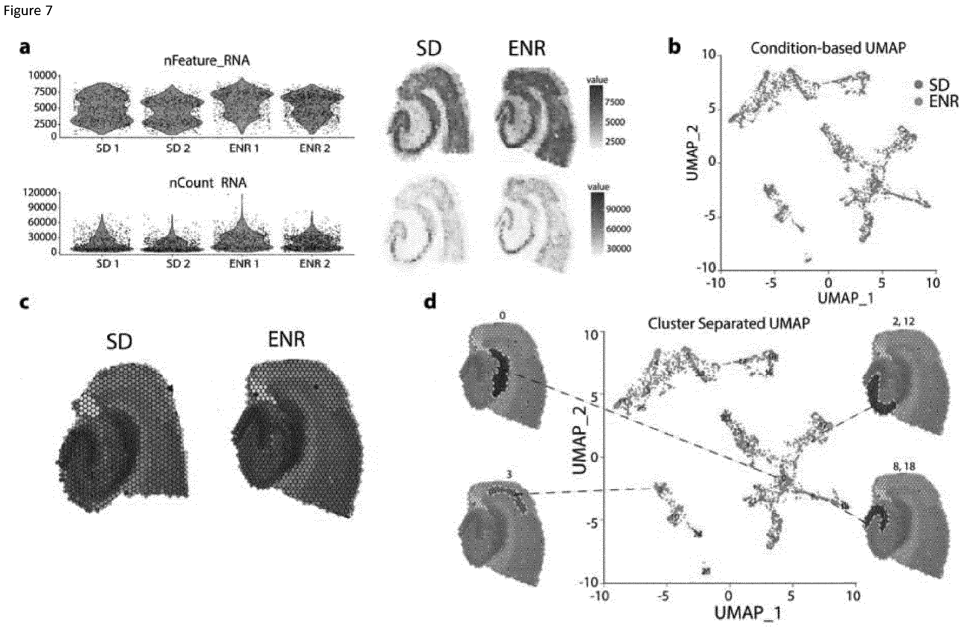

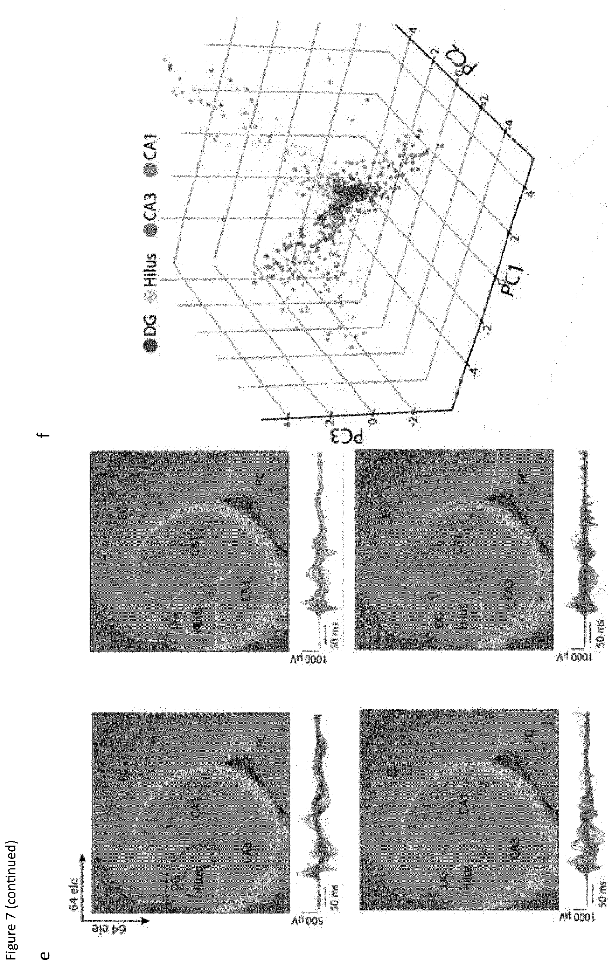

Figure 7 shows in a) the nFeature RNA and nCount RNA statistics, along with feature maps, present the number of distinct genes and the number of unique molecular identifiers (UMIs) in SD (n=2) and ENR (n=2) transcriptomes.

These distributions, part of quality control, highlight consistent counts of unique genes and UMIs across all samples and conditions, effectively mitigating potential technical batch effects and experimental variability, b) Employing both Harmony and Seurat programs, a condition-based UMAP analysis differentiates transcriptomic profiles based on conditions. The resultant UMAP reveals a congruent tissue structure between the two conditions, exhibiting precise cluster separation at high resolution, c) Segmentation of HC clusters based on spatial gene profiles is performed for SD and ENR brain slices, d) Selected clusters, aligned with DG, CA3, CA1, and EC regions, are positioned within a UMAP representation. This lower-dimensional depiction effectively showcases the separation of clusters based on gene expression profiles, capturing tissue heterogeneity and gradual expression variations, e) Through PCA analysis and the K-means algorithm, a classification of multilayered HC oscillatory waveform shapes is established. This classification is rooted in features extracted from LFP events, unraveling intricate insights into the diversity of waveform patterns across different layers of the hippocampus.

Figure 8 in a-f) illustrates the elevated transcriptional expression observed in ENR compared to SD conditions across HC clusters. Genes from six distinct functional families, encompassing Immediate Early Genes (IEGs), Hippocampal Neurogenesis, Signaling Pathways, Receptors and Channels, Synaptic Plasticity, and Synaptic Vesicles, are systematically categorized. Notably, key genes critical for neuronal activity and function, such as *Bdnf*, *Egr1*, *Homer1*, *Npas4*, *Gria2*, and *Campk2a*, display heightened expression levels within the DG transcriptome of ENR, underscoring the enriched transcriptional landscape induced by environmental enrichment ($p < 0.001$ ANOVA).

Figure 9 shows a) an analysis of gene expression interplay matrices within six gene families using mutual information reveals an intriguing contrast between SD and ENR conditions. ENR displays heightened statistical transcriptional connectivity and coordinated activity compared to SD, suggesting a robust influence on HC-subnetwork relationships, b) Cross-covariance of pairwise firing electrodes is evaluated through PCC, unveiling intricate connectivity patterns. Compared to SD, ENR connectivity matrix exhibits amplified local and global spatiotemporal interactions, which harmonize with the cross-correlogram observations. These findings collectively shed light on the nuanced impact of ENR on the dynamic transcriptional-functional interplay within hippocampal subnetworks.

Figure 10 shows in a) that mutual information distance scores were computed for target gene families within SRT spots, revealing differences in interactions within HC subnetworks. The ENR transcriptome exhibits higher mutual information, indicating robust, coordinated activity and communication among gene expression patterns compared to SD ($p < 0.001$ ANOVA). (b) Correlation matrices are quantified using PCC of LFP activity across interconnected HC layers ($p < 0.001$ ANOVA). This corresponds with increased spatial interactions in ENR compared to SD networks, as shown in (a).

Figure 11 shows in a) the application of sparsity-constrained NMF analysis, as demonstrated in Figure 2c-f. b-d) The analysis quantifies decomposed sets of genes, spatial locations, and connectivity node degree features, revealing simultaneous variations in network topological metrics linked to spatially-resolved IEG expression and network connectivity.

Figure 12 shows the application of XGBoost algorithm to predict network electrophysiological metrics (LFP rate, amplitude, and duration) from transcriptomic data of each specified gene family from SD and ENR data. The prediction of n-Ephys metrics from the transcriptomics data is evaluated with Pearson correlation coefficient (r) in SD and ENR. The significant difference between the predicted SD and ENR values in all gene families is indicated (***$p < 0.001$, ANOVA).

## EXAMPLES

The inventive platform has been tested using brain tissues from healthy mouse models. These mice were housed in different conditions, including standard and enriched environments, to study experience-dependent plasticity paradigms. The enriched environment condition is known to promote the enhancement of experience-dependent plasticity through increased stimuli and differentiated social interactions.

The platform was employed to simultaneously capture and integrate molecular and functional information across multiple spatiotemporal scales within these intact brain tissues. This involved high-resolution spatiotemporal recording of functional firing pattern dynamics from large-scale cell assemblies, optical imaging for spatial localization, and high-resolution multiplexed profiling of cellular transcriptomics from the same assemblies in the hippocampal-cortical circuit.

The platform successfully unveiled the potential of causal prediction from spatiotemporal transcriptomics to cellular functional scales. This allowed for the identification of the upstream circuit computational processing and biophysical underpinnings, which is crucial for mapping the molecular identity of large-scale neural circuits.

The development and testing of the platform has already provided valuable insights into the molecular mechanisms underlying functional enhancements induced by different environmental conditions. The platform's adaptability and capabilities hold immense potential for diagnostic tools and therapies for precision personalized medicine based on gene-neural electrophysiology features of individuals.

## Methods

### Animals and Acute Brain Slice Preparation

All experiments were performed on 12-week-old C57BL/6J mice (Charles River Laboratories, Germany) in accordance with the applicable European and national regulations (Tierschutzgesetz) and were approved by the local authority (Landesdirektion Sachsen; 25-5131/476/14). Female C57BL/6J mice were obtained at five weeks of age and randomly distributed into two experimental groups - standard housed (SD) and enriched environment (ENR) housed as previously described (15). ENR-housed mice lived in a specially designed cage containing rearrangeable toys, maze-like plastic tubes, tunnels, housing, and additional nesting material. This ENR cage environment has been shown to promote the enhancement of experience-dependent plasticity through increased stimuli and differentiated social interactions. Mice stayed in the assigned environment for six weeks before the experiments began and remained until their experimental date.

**Acute brain slices were prepared according to the inventor's previous studies (13,16). Briefly,** mice were anesthetized with isoflurane before decapitation. The brain was carefully removed from the skull and placed in a chilled cutting sucrose solution prior to slicing. The brain was placed into a custom-made agarose container and fixed onto the cutting plate. Dorsal, horizontal slices (300 $\mu$m thick) were prepared using Leica Vibratome VT1200S (Leica Microsystems, Germany). Slices were cut at 0-2 °C in an aCSF solution saturated with 95% $O_2$ and 5% $CO_2$ (pH = 7.2-7.4) of a high sucrose solution containing in mM: 250 Sucrose, 10 Glucose, 1.25 $NaH_2PO_4$, 24 $NaHCO_3$, 2.5 KCl, 0.5 Ascorbic acid, 4 $MgCl_2$, 1.2 $MgSO_4$, 0.5 $CaCl_2$. Next, HC slices were incubated for 45 min at 34 °C and then allowed to recover for at least 1 hour at room temperature before being used for network electrophysiology (n-Ephys) recordings with a high-density neurochip. A perfusate solution used during recordings contained in mM: 127 NaCl, 2.5 KCl, 1.25 $NaH_2PO_4$, 24 $NaHCO_3$, 25 Glucose, 1.25 $MgSO_4$, 2.5 $CaCl_2$, and was aerated with 95% $O_2$ and 5% $CO_2$.

### Extracellular n-Ephys Recordings.

All electrical recordings were performed using high-density CMOS-based biosensing MEA chips (CMOS-MEA) and an acquisition system (3Brain AG, Switzerland) customized to the present recording setup. CMOS-MEA are composed of 4096 recording electrodes with a 42 $\mu$m pitch size to compose an active sensing area of ~7 mm$^2$. The on-chip amplification circuit allows for 0.1-5 kHz band-pass filtering conferred by a global gain of 60 dB sufficient to record slow and fast oscillations (13). For extracellular recordings, slices were moved and coupled onto the chip using a custom-made platinum harp placed above the tissue. To minimize experimental variation and maintain slice longevity, a heat-stabilized perfusion system delivered oxygenated recording perfusate to the neurochip with a flow rate of 4.5 mL/min and was temperature controlled at 37°C throughout the experiment and recordings. The inventors collected extracellular recordings at 14kHz/electrode sampling frequency and 1Hz recording frequency from spontaneous network-wide activity through pharmacological-induced evoked responses using 100 $\mu$M 4-aminopyridine (4AP) (16) (Sigma-Aldrich, Germany). All solutions were freshly prepared, and pharmacological compounds were dissolved into the recording perfusate for the experiment. A custom-designed modular stereomicroscope (Leica Microsystems, Germany) was incorporated into the system to capture the acute slice light imaging simultaneously with the whole HC circuit extracellular n-Ephys recordings. During offline analysis, these images were used to maintain the spatial organization of the brain slice tissue relative to the n-Ephys electrode layout.

### Spatially-resolved Transcriptomics in Hippocampal-cortical Slices

Spatially resolved HC sequencing and transcriptomic analysis were performed using SRT Visium gene expression slides (10X Genomics, USA). SRT slides are composed of 4 distinct capture areas containing 5,000 spatially barcoded spots with a 55 $\mu$m diameter to compose a capture area of ~6.5 mm x 6.5 mm sufficient for placement of the entire mouse HC slice. Immediately following n-Ephys recordings, slices were embedded in ~6.5 mm x 6.5 mm Tissue-TEK Cryomold containing Optimal Cutting Temperature (OCT) solution, frozen over dry ice, placed in a WHEATON CryoELITE tissue vial, and stored at -80 °C to maintain tissue viability until SRT experimental date. Here, to optimize the number of cells per spot and provide a clear transcriptomic profile, slices were horizontally cryosectioned to 18 $\mu$m using Thermo Fisher Cryostar NX70 (Thermo Fisher Scientific, USA). Tissue was mounted on the SRT gene expression slide, and methanol fixed at -20°C for 30 minutes. Slices were hematoxylin and eosin (H&E) stained, and bright-field imaged to obtain morphological slice images. Following imaging, the slices were enzymatically permeabilized for 22 minutes on a thermocycler, and the resultant released mRNA was bound to the thousands of spatially barcoded mRNA-binding oligonucleotides within each spot. To generate cDNA from the oligonucleotide-bound mRNA, an enzymatic reverse transcription mixture (10X Genomics, USA) was applied and incubated in a thermocycler at 53°C for 45 minutes. To generate cDNA second-strand synthesis, an enzymatic second-strand mixture (10X Genomics, USA) was applied and incubated in a thermocycler at 65 °C for 15 minutes. To denature enzymes, a basic elution buffer (EB) (Qiagen, Germany) with a pH of 8.7 was applied, and the final sample was stored in a corresponding tube per capture area containing Tris-HCl. Finally, the spatially barcoded, full-length cDNA is prepared for library sequencing through PCR amplification. To determine the optimal cycle number (Cq) via qPCR, a qPCR mix using KAPA SYBR FAST (Kapa Biosystems, USA) and a 1 μl sample from each cDNA sample was added to a clean qPCR plate. Following the incubation protocol, a Cq value of 15.7 was determined for the cDNA sample, which corresponded to 16 amplification cycles. An amplification mixture (10X Genomics, USA) was added to the cDNA sample tubes, and the qPCR amplification protocol was completed according to the obtained Cq value. Samples were stored overnight at 4°C overnight before proceeding to sequencing. Library construction and sequencing were carried out at the Dresden Concept Genome Center (DcGC) using the HiSeq 2000 Next Generation Sequencer (Illumina, Inc., USA).

Sequenced data was processed with the Space Ranger (10X Genomics, USA) pipeline to recreate the spatial arrangement, which aligns the H&E stained bright-field image with the spatially barcoded gene expression data based on the fiducial spots in the slide capture area border. The pipeline performs alignment, tissue detection, fiducial detection, and barcode/UMI counting.

**Data Analysis**

All basic and advanced algorithms used in this work were developed and implemented with custom-written Python scripts. Any package add-ons are cited accordingly.

**SRT Quality Control and Gene Expression Normalization**

Prior to data analysis, technical batch effects and experimental variation were ruled out using a single-cell analysis toolkit, Seurat (58), and an add-on package STutility (https://ludvigla.github.io/STUtility web site/). These packages statistically quantified the number of unique genes (nFeature RNA) and the number of UMIs (nCount RNA) across all samples and conditions. To further delineate and find shared hippocampal structures between the two conditions, a further add-on package, Harmony, recomputed the UMAP embedding and clustering to return an integrated low-dimensional representation of the data (59). As each dataset was found to have ~5500 median genes per spot, SRT spots in each dataset with fewer than 1000 unique genes were filtered out of the analysis. Next, to downsize the total number of genes for analysis, mitochondrial and ribosomal protein-coding genes were filtered out of analysis. Finally, to account for technical batch variation and detect highly variable genes, overall gene expression per SRT spot was normalized by total counts of each gene over all SRT spots so that each spot has the same count after normalization.

This was implemented using the scanpy.pp.normalize_total python package and is available on GitHub (https://github.com/theislab/scanpy) (60).

**Oscillatory Pattern Detection and Waveform Classification**

Prior to data analysis, oscillatory patterns of LFPs were detected in each recording with hard threshold algorithms (16). Furthermore, detected events were further processed and filtered with a low-pass 4th-order Butterworth filter (1-100Hz).

Finally, quantile thresholding was employed in a custom-written Python script to remove spuriously firing electrodes or non-physiological detected events (16). To characterize and allocate the distinct features and shapes of the recorded LFP oscillatory waveforms to specific interconnected HC layers, the inventors implemented Principal Component Analysis (PCA) and K-means clustering algorithms in a procedure as previously described (13).

**Structural Clusters**

To characterize local and global hippocampal subnetwork behavior, the functional firing n-Ephys electrodes were structurally related to a specific HC region through an overlay of light microscope hippocampal images on the CMOS-MEA layout. Electrodes were then grouped into clusters based on structural markers on the HC slice - DG, Hilus, CA1, CA3, EC, and PC (16). To characterize the transcriptomic profile in these six major regions, SRT spots were structurally related to a specific HC region through an overlay of H&E-stained bright field microscope images on SRT spot layout using Loupe Browser (10X Genomics, USA).

**Multiscale Spatial Alignment**

To infer a correspondence between the n-Ephys electrode-SRT spot interface and their respective network-wide functional electrical activity and transcriptomic feature readouts with spatial localization, MEA-seqX implements a multiscale spatial alignment procedure. To provide the transcriptomic and electrophysiologic profiles of the same cell assembly with spatial context, automatic slice alignment is performed using image resizing and rotation. This alignment is based on optical imaging, n-Ephys electrode-SRT spot interface physical size, and related hippocampal-cortical structural inputs to put the multiscale data in the same dimension. First, MEA-seqX implements an automatic scaling algorithm based on n-Ephys electrode-SRT spot sizes to resize the light H&E-stained bright field microscope slice image from SRT to the respective light microscope hippocampal image from n-Ephys. Importantly, the n-Ephys electrode-SRT spot matching is not one-to-one due to the difference in technology resolution; instead, it is a fractional matching based on related hippocampal-cortical structural inputs. As such, each SRT spot has related n-Ephys electrodes with averaged electrophysiological features from the related electrodes. Next, slice spatial alignment and rotation are computed between the two slice images with the following procedure - i) Hippocampal-cortical structural reference points $\{i,j\}$ and $\{k,l\}$ are assigned for each scale, SRT and n-Ephys respectively, where $\{i,k\}$ are midpoints in the DG crest and $\{j,l\}$ are on the DG supra blade edges, ii) SRT reference point $i$ is aligned with n-Ephys reference point $k$ to place both scales in one dimension, iii) Following the alignment of reference points $\{i,k\}$, a final alignment for reference points $\{j,l\}$ is based on the difference between $\Theta_{n\text{-}Ephys}$ and $\Theta_{SRT}$. iv) Given that the coordinates of both arrays are known, the distance of $x$, $x'$, $y$, and $y'$ is used to calculate the angle of $\Theta_{SRT}$ and $\Theta_{n\text{-}Ephys}$. v) To determine $\Theta_{SRT}$ the horizontal intersection line between aligned reference points $\{i,k\}$ was used to define x while a vertical intersection line between reference point j and the horizontal intersection line was used to define y. vi) To determine $\Theta_{n\text{-}Ephys}$, the horizontal intersection line between aligned reference points $\{i,k\}$ was used to define x' while a vertical intersection line between reference point $l$ and the horizontal intersection line was used to define $y'$. vii) The final angle of rotation is defined as $\Theta_{iNeuromics} = \Theta_{n\text{-}Ephys} - \Theta_{SRT}$ which, when applied, align reference points $\{i,k\}$ as the final multiscale reference point m and align reference points $\{j,l\}$ as the final multiscale reference point $n$.

**Mean Activity n-Ephys Features**

To determine how spatial gene expression patterns are related to a functional n-Ephys feature, filtered genes were correlated with one of the network features using Spearman's correlation (61) and sorted according to significance using Benjamini-Hochberg false discovery rate adjusted p-value (62). Functional network features of large-scale spatiotemporal LFP oscillations included LFP rate, amplitude, energy, delay, and positive and negative peak count (13,16).

**Targeted Gene Lists**

Specific gene lists were formulated based on functional gene ontologies. Genes related to families of immediate early genes, signaling pathways, hippocampal function, and neurogenesis were compiled into six lists (24,25).

**Non-Negative Matrix Factorization (NMF)**

The inventors implemented an unsupervised machine learning algorithm using a sparsity-constrained non-negative matrix factorization to identify individual spatiotemporal patterns emerging from SRT and n-Ephys networks (26). NMF factorization is described and adapted from the Scikit-learn 1.2.2 python package (sklearn.decomposition.NMF) (63). First, the input $V$ matrix contains the combined information from SRT (i.e., gene expression values from the IEG gene family) and n-Ephys (i.e., LFP rate or Degree network feature), with each data entry comprising the expression value of each gene related to network feature value (n) with spatial localization (m).

$$V \approx \mathrm{WH},\ V = n\ x\ m,\ W = n\ x\ p,\ H = m\ x\ p$$

Where $n$ is the spatially localized spots, $m$ is the related gene expression to network feature, and $p$ is the number of factors.

The resultant decomposed basis matrix W contains the spatial gene expression patterns and their locations related to the functional feature, and the coefficient matrix H represents the contribution of these basis vectors to each spatial location and n-Ephys feature. To optimize the distance between $V$ and the product matrices $H$ and $W$, the inventors implemented the widely used distance optimizing function squared Frobenius norm (F), which added sparsity constraints for the factors (63).

$$\min_{W,H}\left\{\|V-WH\|_F^2 + \alpha\|W\|_F^2 + \beta\sum_{j=1}^{y}\|H(:,j)\|_1^2\right\}$$

where $W_{ij}$ and $H_{jj}$ are nonnegative value ($1 \leq i \leq x$, $1 \leq j \leq y$). $\alpha$ and $\beta$ are the corresponding regularization parameters for $H$ and $W$.

**Mutual Information**

To present the collectivity of spots in a multilayered network based on gene expression, gene expression distribution was calculated for each target gene list. Next, mutual information distance scores were calculated for the gene information from each target gene list in each spot, compared between spots, and sorted by cluster (27). The inventors computed the mutual information using adapted functions from Scikit-learn Scikit-learn 1.2.2 python package (sklearn.metrics.normalized_mutual_info_score) (63).

**Functional Connectivity**

To infer large-scale statistically dependent connectivity for functional firing activity over a multilayered hippocampal network, cross-covariance was calculated between pairs of firing n-Ephys electrodes using Pearson's correlation coefficient (PCC) followed by Directed Transfer Function (DTF) and Multivariate Granger causality as previously described (13,16).

**Graph Map Visualization**

To visualize large-scale network connectivity in both SRT and n-Ephys datasets, the mutual information distance scores and functional connectivity data architecture, respectively, were constructed to contain nodes and edges as described previously (16). The data were converted into (.gexf) file format and were directly read and visualized in the Gephi program 9.2 version (https://gephi.org). To study the functional interactions of selected genes over the spatial network array, the inventors filtered the mutual information scores between all paired spots to include those over the mean and two standard deviations. Therefore, the threshold value was set to include genes with $r \geq 0.8$ for each target gene list. To examine the functional connections of n-Ephys electrodes, the top 2% of the total functional links were included. Both SRT and n-Ephys connectivity maps were plotted with similar edge weights and degree range queries.

**Network Topological Metrics**

Graph Theory was used to characterize overall network topology and interconnectedness based on functional connectivity from n-Ephys detected LFP events or mutual information scores from SRT gene expression. The inventors computed the topological metrics in custom-written Python code, as the inventors previously reported (13,16). Briefly, the network connectivity topological metrics are described by considering the node *n* as the central component of the **graph that may or may not be connected to one another. In the inventor's case, a node *n*** corresponds to a specific n-Ephys electrode or SRT capture spot in the sensing arrays, where the edges *e* are the functional links or connections between each node *n*. To present overall network topology and features, the inventors selected the following graph theory topological parameters:

**Degree**

To characterize the different representations of network connectivity, the inventors characterized the degree k of a node n to describe the number of edges connected to a node as previously described (16).

$$k_i = \sum_{j\in N} a_{ji}$$

where $k_i$ denotes the degree of a node $i$. $a_{ji}$ denotes the connection between nodes *i* and *j*. *N* is the set of all computed nodes in the network.

**Hub Nodes and Rich Club Nodes**

To determine centralized, important nodes in a network and reveal network topology, hub nodes, and rich club nodes were analyzed. Hub nodes were detected based on three nodal metrics - node strength, clustering coefficient, and network efficiency. The metric value for each node was calculated and compared to determine whether the node value was in the top 20% of all nodes (16). To restrict the definition of the hub node, the inventors set limits with a **hub score. The inventor's hub score was valued between** 0 and 3, where nodes either satisfied the top 20% in none, 1, 2, or all 3 nodal metrics. Within the hub node group is a subgroup of nodes with dense connections that conferred the rich-club nodes and are described as hub nodes with a higher degree than the average and provided by the rich club coefficient *ϕ(k)* (16)

$$\phi(k) = \frac{2E_{>k}}{n_{>k}(n_{>k} - 1)}$$

Where $k$ denotes the degree, $n_{>k}$ represents the number of nodes whose degree is larger than a given value $k$, and $E_{>k}$ denotes the number of connections in a subnetwork comprising $n_{>k}$.

**Network Topology Characterization**

To determine the potential impact of hub nodes on the network function and the organizational processes shaping network topology, the inventors characterized the degree distributions P(k) of detected nodes in n-Ephys and SRT datasets, which resulted in decayed distribution with a power-law tail (64).

$$P(degree = k) \sim k^{-\alpha}$$

To estimate the power-low degree distribution $P(k)$ to describe the scale-free topology with a small-world attribute, the inventors used the lognormal model fit.

$$P(k) = \frac{1}{\sigma\sqrt{2\pi}} e^{-\frac{(k-\mu)^2}{2\sigma^2}}$$

Where $\mu$ and $\sigma$ are the mean and standard deviation of the distribution, respectively. To visualize the best-fit network characterization, a complementary cumulative distribution function (cCDF) was used instead of the probability density of the node degree and plotted on logarithmic axes for a more robust visualization of the high-k regime. Goodness-of-fit tests were performed between actual data and fitted models and were estimated by the coefficient of determination $R^2$. Finally, Pareto linear binning (scipy.stats.pareto) (65) was applied to discretize the power law distribution.

**Diffusion Pseudotime**

To pinpoint dynamic transcriptional changes from static, spatially resolved sequencing data and to determine the impact of intrinsic and extrinsic influences on the distinct dynamic process under examination, the inventors employed diffusion pseudotime (DPT) (35). DPT uncovers the underlying dynamics of biological processes and, in this case, the temporal trajectories of specific gene expression from spatially resolved hippocampal transcriptomes. While traditional diffusion maps effectively denoise data while maintaining the local and global structure, the resultant maps usually encode the information in higher dimensions, limiting the visualization. To overcome this prior to the employment of DPT analysis, the inventors implemented the potential of heat diffusion for affinity-based transition embedding (PHATE) on spatial transcriptomic data, which presents information at lower dimensionality (https://github.com/KrishnaswamyLab/PHATE) (66). PHATE encodes both local data and global data in a manifold structure. Local data relationship similarities are encoded by applying a kernel function on Euclidean distances. Global data relationships are encoded via potential distances where the local similarities are transformed into probabilities. These diffusion probabilities are determined by transforming the local information into the probability of transitioning from one data point to another in a single step of a random walk. This can be powered to t-steps to give t-step walk probabilities for both local and global distances. In the **inventor's dataset, each spatial spot has a determined relationship to each nearest neighbor or** distant spot in a weighted graph (66). DPT analysis then orders the transcriptomic spots according to the probability of differentiation toward a different spot (35).

**Cell-Type Deconvolution**

To determine cell type colocalization and examine differences between two hippocampal transcriptomes, conditional autoregressive-based deconvolution (CARD) was performed using a single-cell sequencing reference (39,40). The CARD-based analysis is found on GitHub (https://github.com/YingIVla0107/CARD) and was adapted for spatial transcriptomic data in Python. Within the reference was a broad group classification of hippocampal cell types, including astrocytes, endothelial cells, ependymal cells, macrophages, microglial cells, neurogenic cells, neurons, oligodendrocytes, and polydendrocytes with the accompanying subgroups. Low-count cell type filtering downsized 85 to 76 hippocampal cell types.

**Prediction with XGBoost Algorithm**

To determine if specific gene expression values can predict related HC network feature parameters per SRT spot, such as LFP rate, amplitude, and duration, the inventors implemented the Gradient Boosting (XGBoost) Algorithm, which integrates multiple tree models and has a strong interpretability (47). XGBoost is described in the Scikit-learn 1.2.2 Python package (sklearn. ensemble. GradientBoostingClassifier) with training and testing datasets implemented as described in the package (sklearn.model_selection.train_test_split) (63). These datasets contained the spatially resolved gene expression values based on specific gene lists and the related network features from functional n-Ephys datasets. To level input information between the SD and ENR datasets for better comparison, half of the ENR dataset was randomly generated to use as input for the training and testing datasets. This normalization was selected as the ENR has a two-fold difference in network feature parameters, as previously described (16). The input datasets were split equally into an initial training dataset and a testing dataset, real data points trained over 100 iterations. The final predicted and real data points were used to determine the predictability, accuracy, and significance of network feature prediction from transcriptomic data (67). Packages implemented for statistics included Scikit-learn 1.2.2 Python packages to calculate the prediction accuracy (sklearn.metrics.explained_variance_score) (63) and Scipy 1.10.1 to calculate the Pearson correlation coefficient (scipy.stats.pearsonr) and (scipy.stats.ttest_ind) (65) . Accuracy results were compared over multiple final data outputs and values over the mean, and three standard deviations were determined to be within the threshold of chance.

**Statistical Analysis**

All statistical analysis was performed with Originlab 2020 or as described in package add-ons. Data in this work were expressed as the mean $\pm$ standard error of the mean (SEM) unless otherwise denoted as standard deviation. Box charts are determined by the 25 th-75 th percentiles and the whiskers by the 5 th-95 th percentiles with lengths within the Interquartile range (1.5 |QR). Also, the lines depict the median and the squares for the mean values. Differences between groups were examined for statistical significance, where appropriate, using the Kolmogorov-Smirnov test, one-way analysis of variance (ANOVA), or two-way ANOVA followed by Tukey's posthoc testing. $P < 0.05$ was considered significant.

**Results**

**Interfacing Technologies and Integrating Information** - **from Transcriptome to Functional Networks**

MEA-seqX integrates brain-on-chip technology via network electrophysiology (n-Ephys) of high-density CMOS-microelectrode array (CMOS-MEA) (13-16), bioimaging via optical microscopy, and spatial sequencing technology via spatially resolved transcriptomics (SRT) (Figure 1a-d). Specifically, high-resolution spatiotemporal recordings of extracellular firing patterns obtained from 300 $\mu$m mouse hippocampal-cortical 'HC' acute slices interfaced with 4,096-on-chip sensors. Simultaneously, optical imaging was employed for precise anatomical localization, and high-resolution transcriptomic profiling data was obtained from the same cell assemblies within the circuit (Figure 1e).

**Quality control of the inventor's transcriptomic datasets revealed similar nFeature and nCount**

RNA statistics and tissue structure when visualized via a Uniform Manifold Approximation and Projection (UMAP) method (22) (Figure 7a-c). Network-wide activity in the HC was assessed through Principal Component Analysis (PCA) and K-means clustering algorithms (13), which provided distinct features of oscillatory waveforms and their shapes in each interconnected HC layer (Figure 7d). The multidimensional readouts were processed through a Python-based computational pipeline to quantitatively map the molecular dynamics of the circuit at high spatiotemporal resolution.

A multiscale spatial rescaling and alignment procedure was developed to establish a direct correspondence between the n-Ephys electrode-SRT spot interface and their respective network-wide functional electrical activity and transcriptomic feature readouts with their localization in the tissue.

This was implemented with automatic scaling algorithms, employing image resizing and rotation based on optical imaging, n-Ephys electrode-SRT spot interface physical size, and two anatomical landmarks in the dentate gyrus (Figure 1f; for details, see Methods). The resulting overlay allowed the identification of network features from the transcriptomics readouts in alignment with readouts of neural activity (Figure 1g). The method according to the present invention generated precise topological graphs of multimodal data connectivity, presenting both the local and global relationships of SRT spots and the underlying firing electrodes (Figure 1h). From the wealth of high-dimensional data, transcriptional pseudotime dynamics underlying firing information flow were derived (Figure 1i). By integrating a deconvolution method, the inventors inferred cell-type resolution from the spatial transcriptomic data and correlated the neuronal heterogeneity to their firing features (Figure 1j). Finally, thod according to the present inventionprovided an automatic machine-learning algorithm to predict high-accuracy network electrophysiological features from spatial transcriptomic profiles (Figure 1k).

**Linking Spatial Transcriptome to Network-wide Neural Functional Dynamics**

To apply the pipeline to a concrete, previously unsolvable research question, the inventors used the MEA-seqX framework to uncover the impact of experience-dependent plasticity (16) on the coordinated activity of neuronal ensembles and their interaction with orchestrated transcriptional activity. HC slices from mice housed in standard (SD) and enriched environment (ENR) conditions were prepared for recording oscillatory patterns of local field potentials (LFPs), optical imaging, and SRT sequencing. To assess how spatial patterns of gene expression (SRT) correspond to the functional network electrophysiological features (n-Ephys) of the same brain tissue, the inventors measured Spearman's correlation (23) to quantify the transcriptional similarity of gene expression profiles from SRT spots and to examine their relationship to functional network features (i.e., amplitude, delay, energy, LFP rate, negative peak count, and positive peak count). The inventors found a significant enhancement in the gene expression pattern corresponding to the functionally coupled dentate gyrus (DG) and CA3 regions in ENR compared to SD (Figure 2a). In particular, the LFP rate showed a 2.2-fold, 2.5-fold, and 0.6-fold increase of correlated transcripts in the DG, CA3, and CA1 subfields, respectively, in the ENR compared to the SD (Figure 2b).

The inventors next sought to identify which specific genes would drive a stronger causal link between correlated molecular and functional networks. Based on gene ontology clusters, the inventors categorized correlated genes into six targeted gene families, including Immediate Early Genes "IEGs", Hippocampal Neurogenesis, Hippocampal Signaling Pathway, Receptors and Channels, Synaptic Plasticity, Synaptic Vesicles, and Adhesion (24,25). Examining transcripts from these families illustrated enhanced expression of IEGs, ion channel activity, synaptic function, and neurogenesis in ENR than SD. Genes essential for hippocampal activity and function, such as *Bdnf, Egr1*, *Homer1*, *Npas4, Gria2*, and *Campk2a*, had higher expression levels in the hippocampal transcriptome of ENR compared to SD (Figure 8).

Next, to identify distinct spatiotemporal patterns across the combined SRT and n-Ephys modalities, the inventors implemented an unsupervised machine learning algorithm using a sparsity-constrained non-negative matrix factorization (NMF) (26) This approach allowed decomposing modalities into sets of differentially expressed subnetworks of genes, spatial locations, and electrophysiological features to provide dimensionality reduction and interpretability (Figure 2c). As IEGs exhibited a significant contribution to linking spatial transcription patterns to LFP activity (Figure 8), the number of factors in the NMF decomposition was determined based on the size of the IEGs list (i.e., 12 factors). The input expression V matrix, which contains the combined information from SRT and n-Ephys (i.e., LFP rate) data, with each entry representing the expression level of a gene correlated to network function feature at a specific spatial location (Figure 2d), was decomposed into two non-negative matrices. The basis matrix W contains the spatial gene expression patterns and their locations rated to the functional feature (Figure 2e), and the coefficient matrix H represents the contribution of these basis vectors to each spatial location and n-Ephys feature (Figure 2f). This analysis unveiled increased normalized gene expression and network feature values in the ENR network, along with higher spatially resolved components driving the strength in gene expression. Additionally, the inventors found spatially specific subnetworks in the SD and ENR networks, highlighting genes such as *Bdnf, Egr1*, *Fosb,* and *Npas4*.

MEA-seqX suggests a computational role of the experience-dependent dynamics in the coordinated interaction of neuronal ensemble activity and their corresponding transcription patterns.

**Coordinated Topological Network Organization of Spatially-resolved Transcriptome and Activity Patterns**

The inventors employed quantitative measures to comprehensively examine the interconnections between HC subnetworks derived from SRT data and neural n-Ephys recordings under SD and ENR conditions. The inventors calculated 'mutual information' (27), i.e., the measure of the mutual dependence between two variables, in order to assess the extent of the interdependence of gene expression within specific gene families (Figure 9a) and Pearson's correlation coefficient (PCC) (13,16) to gauge the cross-covariance among pairs of firing electrodes (Figure 9b). These computations allowed the inventors to establish connectivity matrices on multiple scales. Next, the inventors computed mutual information distance scores for each target gene family in every spot to gauge the dissimilarities in interaction within the different spots. These scores were then compared between spots and organized into clusters (27) (Figures 10a-f). Simultaneously, the inventors quantified differences in the correlation matrices by analyzing the PCC of concurrent LFP activity across interconnected HC layers (13,16) (Figure 10g). Upon analyzing various gene families, it became evident that the ENR transcriptome, in contrast to SD, exhibited higher mutual information. This suggests a more robust statistical relationship in coordinated activity and communication among gene expression patterns within the HC subnetworks (Figure 10a-f). Importantly, as evident from the LFP cross-correlogram16 displayed in (Figure 10g), this finding strongly aligned with the significant enhancement in both the local and global strength of spatiotemporal interactions in ENR vs. SD networks (16).

Subsequently, in order to describe and quantify the topological organization, flow of information, and communication properties within the multimodal transcriptional-functional readouts, MEA-seqX employed a graph-theoretic approach to assessing multiscale network metrics (Figure 3a). This involved constructing detailed wiring diagrams depicting both local and global interconnections between transcriptomics and neural activity patterns. The transcriptomic graph was formed using mutual information scores, while the functional graph was generated from the connectivity patterns among co-firing neuronal ensembles, as captured by the LFPs under SD and ENR conditions. The resultant connectivity maps between transcriptomics and neural function revealed the spatial arrangement of interconnected subnetworks derived from spatial IEGs and spatiotemporal functional neural activity. Remarkably, these maps demonstrated analogous spatial distributions and connectivity patterns across different scales and modes (Figures 3b-c). Comparing HC networks between SD and ENR further emphasized a heightened level of across-scale causal coordination. The transcriptional-functional connectome was enhanced in ENR compared to SD.

The inventors also conducted analyses on the constructed graphs to identify highly interconnected nodes termed "hub complexes" and densely connected hubs referred to as "rich-club organization" within the transcriptional-functional connectomes (28). ENR subnetworks, derived from both SRT and n-Ephys data, exhibited greater interconnected hub complexes and rich-club organization (Figures 3d, e; dark gray) compared to their SD counterparts (Figures 3d, e; gray). This indicates that enriched experience leads to enhanced specialization in coordination, interactions, heightened resilience, and an increased capacity for global communication29 across transcriptional-functional scales. These results shed new light on the dynamic interactions and mutual influences between molecular and functional hub complexes, contributing significantly to the overall coordination of multiscale topological network organization.

Many biological networks are characterized by a small-world topology (30) defined by a scale-free architecture consisting of highly connected hub nodes and a degree distribution that decays with a power-law tail (31). By analyzing cumulative degree distributions of interconnected links in the transcriptomics and functional connectome from SD and ENR networks, the inventors found that these multiscale distributions indeed followed a power-law function, which the inventors have previously also postulated for a transcriptomic network of adult hippocampal neurogenesis (32) and network-wide activity in ENR (16). Both SRT and n-Ephys distributions in ENR networks displayed a heavier tail than SD, indicating a more significant number of densely linked hubs (Figure 3f and insets). This finding is supported by sparsity-constrained NMF analysis of multiscale degree distributions (i.e., similar to those implemented in Figure 2c-f). The inventors quantified multiscale decomposed sets of variably expressed subnetworks of genes, spatial locations, and the network's connectivity node degree features to identify the variation in network topological metrics concomitant with the expression of spatially-resolved IEGs and the degree of network connectivity (Figure 11).

By revealing experience-induced hippocampal connectomics across scales and its intricate **multilayer dynamic characteristics within a single experiment, the inventor's results** demonstrate the capacity of MEA-seqX to integratively capture coordinated transcriptomics and functional data. This integration allows novel insights into neural communication, resilience levels, hierarchical organization, and specialization across multiple scales that previously could only be studied independently and usually based on limited data (29,33,34).

## Assessing Multiscale Dynamics of Multimodal Information

To address the challenge of unraveling the synchronous dynamics across scales and modalities, the inventors combined two cutting-edge computational methods - Diffusion Pseudotime (DPT) (35) for SRT and Center of Activity Trajectories (CAT) (13,16) for n-Ephys. This integrative approach aimed at unraveling the temporal progression of gene expression and network-wide neural activity in hippocampal circuitry.

The inventors applied DPT to the static snapshot SRT data to achieve pseudotemporal ordering by assembling the spots according to expression similarity. This allowed the inventors to construct a network representation of SRT developmental trajectories. The probability of differentiation was computed through Euclidian distances from vector-based randomized distances in the diffusion map space, which facilitated the identification of low-dimensional changes from high-dimensional observations. The inventors here focused on IEGs expression in SD and ENR, revealing significant regional differences in DPT based on IEGs expression (Figures 4a, b). Concurrently, the inventors quantified the intra-hippocampal spatiotemporal propagation pathway by constructing CATs for all n-Ephys circuit-wide oscillatory activity and thereby calculated the rate of spatiotemporal displacement of those firing patterns (Figures 4c, d). The analysis of CATs duration demonstrated faster propagation (i.e., shorter duration) of firing events in ENR compared to SD, as previously reported (16). Remarkably, the comparison of SD and ENR transcriptomes indicated a faster DPT in all four hippocampal regions of ENR transcriptome compared to SD as well, thus mirroring the faster spatiotemporal propagation patterns observed in the ENR obtained from n-Ephys CATs16 (Figures 4c-e). By integrating the DPT spatial maps with the temporal progression of neural activity trajectories, modulated by the impact of rich experience, allowed the inventors to instantiate a multiscale perspective of how molecular and electrical processes unfold simultaneously and interact within a biological system. Diving deeper into experience-dependent activity dynamics, the comparison between SD and ENR becomes particularly enlightening. Such a comparison potentially unravels the causal link between dynamical changes at the molecular level and those on functional scales. Different experiential environments, as represented by SD and ENR, induce distinct responses at the transcriptomic level, which are reciprocally and indivisibly linked to the neural activity in the same cellular assemblies. These differential activity patterns connect transcriptomics to the function and back (36,37).

**Spatiotemporal Cell-type Identification**

Next, to understand the transcriptional diversity of neural cell types and their roles in the firing patterns of the hippocampal circuits (38), the inventors employed the conditional autoregressive-based deconvolution (CARD) method using a single-cell sequencing reference (39,40). This allowed the inventors to determine cell types and local tissue composition from the deconvolved gene expression patterns in order to construct multiscale spatial maps of the heterogeneity of neural types and their firing characteristics in the same hippocampal-cortical tissue. The initial application of CARD yielded a broad group classification of hippocampal cell types. This diverse group encompassed astrocytes, endothelial cells, ependymal cells, macrophages, microglial cells, neurogenic cells, neurons, oligodendrocytes, and NG2 cells. Prior to any filtering, the inventors identified 85 different cell types, a finding that underscored **the experimental validity of the inventor's slice acquisition technique. Following the removal** of low-count cell types, the inventors were left with a robust group of 76 cell types.

Interestingly, when these cell types were exposed to two different transcriptomic inputs from SD and ENR, the inventors observed a consistent distribution of prominent cell types across both transcriptomes (Figure 5a). This result underscores the robustnes**s of the inventor's approach and the reproducibility of the inventor's findings.**

Further, integrating the CARD method into MEA-seqX proved instrumental in achieving spatially resolved cell-type composition and linking it to large-scale oscillatory electrophysiological features (Figure 5b). The inventors identified specific high-proportionate cell types within the DG and CA3 regions based on their unique marker genes. In the DG, granule cells (GCs) were characterized by *Cck* and *Penk* expressions, while CA3 pyramidal cells displayed *Nos1* and *Inhba* markers. In accordance with prior studies (41), the inventors have observed the presence of both common *Cck*-expressing and less frequent *Penk*-expressing GCs within the molecular layers of the DG and suprapyramidal blade (Figure 5c). Notably, the expression of both marker genes was significantly higher levels in ENR compared to SD (Figure 5e).

When these markers were superimposed onto the DG-functional network data, the ENR samples showed enhanced firing patterns and signal amplitude, especially within the DG's suprapyramidal blade (Figures 5c,e). Prior research has associated Penk with enrichment in DG engram cells and its involvement in hippocampal-associated behaviors (41), while Cck is implicated in the dynamic selection and control of cell assemblies in DG (42). **The inventor's** data align with existing reports and highlight a marked increase in DG excitability and temporal dynamics in ENR, suggesting a potential avenue for exploring how enriched experiences might affect the transcriptional-functional interplay in hippocampal circuitry, aiding in understanding the cellular and molecular basis of memory (43). Conducting a similar analysis, focusing on the spatial distribution of the pyramidal cell layer in CA3 (Figure 5d), the inventors identified *Nos1* and *Inhba* marker genes exhibiting significantly higher expression in ENR than SD (Figure 5f). This transcriptomic readout matches the increased LFP rate and distinctive waveform characteristics identified within the CA3 region (Figures 5d,f). *Nos1* has established associations with pivotal neural mechanisms, encompassing long-term potentiation (LTP), synaptic plasticity, and regulating neural circuit dynamics (44,45), while *Inhba* is linked to neuroprotection and neuronal survival (46). These reports provide robust substantiation for the **inventor's enhanced transcriptional**-functional findings within the framework of the experience-dependent paradigm evident in the ENR group. This, in turn, paves the way for a more profound exploration of the specific role played by these marker genes within CA3 pyramidal neurons, along with its potential implications for comprehending neural function and dysregulation across scales.

**Prediction Across Scales and Modalities**

To investigate whether the expression profile of individual spatially-resolved genes can predict HC network-wide electrophysiological features, the inventors employed the Gradient Boosting (XGBoost) Algorithm, known for its strong interpretability by integrating multiple tree models (47). While previous methods have focused on gene properties correlated with electrophysiological and morphological diversity across cell types using low-resolution transcriptomics and electrophysiology (e.g., single-cell RNA sequencing and patch-clamp) (4,5,38), the experiments underlying the present invention aimed at assessing whether specific electrophysiological features could be predicted using spatially-resolved transcriptomic data. The XGBoost model was trained for each quantitative n-Ephys measure using 70% of spatial transcriptomic data points in the detected SRT spots (i.e., 333 genes across the spatial context of HC tissue and six gene families as input). Three spatiotemporal n-Ephys metrics (LFP rate, amplitude, and time delay) were successfully predicted based on the differential spatial gene expression. The relationship between cross-validated predictions and the ground truth was evaluated with the Pearson correlation coefficient (r) for SRT-spot-n-Ephys electrodes (Figure 6a, and Figure 12). By implementing the XGBoost on specific gene families that exhibited higher expression in ENR compared to SD circuits, the inventors observed significantly higher prediction accuracy for the ENR dataset. The XGBoost classifier achieved ~93% accuracy for the ENR dataset and ~70% for the SD dataset (Figure 6b). Such predictive interplay between individual genes or gene families at the transcriptomic level and network-level functionality might support the idea that brain functions are orchestrated via multiscale networks that follow fundamental organizational tenets (18). These results underscore a multiscale causative association between neural activity, plasticity, and distinct spatial gene expressions within specific gene families.

This aligns with the predictions of network-level functionality modulated by prior experience (16). This approach could shed light on critical genes and cellular pathways shaping neuronal responses and overall brain function while revealing the regulatory mechanisms governing **neural dynamics, plasticity, and disease pathogenesis. The inventor's integrated approach** massively reduces experimental complexity, thus enhancing result interpretability and offering guidance for subsequent investigations.

**References as cited**

1. Frackowiak, R. and Markram, H. The future of human cerebral cartography: A novel approach. Philosophical Transactions of the Royal Society B: Biological Sciences 370, (2015).
2. Parent!, I., Rabaneda, L. G., Schoen, H. and Novarino, G. Neurodevelopmental Disorders: From Genetics to Functional Pathways. Trends Neurosci 43, 608-621 (2020).
3. Palop, J. J. and Mucke, L. Network abnormalities and interneuron dysfunction in Alzheimer disease. Nat Rev Neurosci 17, 777-792 (2016).
4. Cadwell, C. R. et al. Electrophysiological, transcriptomic and morphologic profiling of single neurons using Patch-seq. Nat Biotechnol 34, 199-203 (2016).
5. Fuzik, J. et al. Integration of electrophysiological recordings with single-cell RNA-seq data identifies neuronal subtypes. Nat Biotechnol 34, 175-183 (2016).
6. Li, Q. et al. Multimodal charting of molecular and functional cell states via in situ electro-sequencing. Cell 186, 2002-2017.e21 (2023).
7. Stähl, P. L. et al. Visualization and analysis of gene expression in tissue sections by spatial transcriptomics. Science (1979) 353, 78-82 (2016).
8. Vickovic, S. et al. High-definition spatial transcriptomics for in situ tissue profiling. Nat Methods 16, 987-990 (2020).
9. Maniatis, S. et al. Spatiotemporal dynamics of molecular pathology in amyotrophic lateral sclerosis. Science (1979) 364, 89-93 (2019).
10. Asp, M. et al. A Spatiotemporal Organ-Wide Gene Expression and Cell Atlas of the Developing Human Heart. Cell 179, 1647-1660.el9 (2019).
11. Urai, A. E., Doiron, B., Leifer, A. M. and Churchland, A. K. Large-scale neural recordings call for new insights to link brain and behavior. Nat Neurosci 25, (2021).
12. Buzsäki, G. Large-scale recording of neuronal ensembles. Nat Neurosci 7, 446-451 (2004).
13. Hu, X., Khanzada, S., Klütsch, D., Calegari, F. and Amin, H. Implementation of biohybrid olfactory bulb on a high-density CMOS-chip to reveal large-scale spatiotemporal circuit information. Biosens Bioelectron 198, 113834 (2022).
14. Amin, H., Nieus, T., Lonardoni, D., Maccione, A. and Berdondini, L. Sei. Rep. , , 2460. 7, (2017).
15. Imfeld, K. et al. Large-scale, high-resolution data acquisition system for extracellular recording of electrophysiological activity. IEEE Trans Biomed Eng 55, 2064-73 (2008).
16. Emery, B. A., Hu, X., Khanzada, S., Kempermann, G. and Amin, H. High-resolution CMOS-based biosensor for assessing hippocampal circuit dynamics in experience-dependent plasticity. Biosens Bioelectron 115471 (2023) doi: 10.1016/J.BIOS.2023. 115471.
17. Emery, B. A. et al. Large-scale Multimodal Neural Recordings on a High-density Neurochip: Olfactory Bulb and Hippocampal Networks, Ieee Embs 42-45 (2022) doi:10.1109/EMBC48229. 2022. 9871961.
18. Luo, L., Callaway, E. M. & Svoboda, K. Genetic Dissection of Neural Circuits: A Decade of Progress. Neuron 98, 256-281 (2018).

19. Buzsäki, G. Large-scale recording of neuronal ensembles. Nat Neurosci 7, 446-451 (2004).

20. Panzeri, S., Macke, J. H., Gross, J. and Kayser, C. Neural population coding: Combining insights from microscopic and mass signals. Trends Cogn Sei 19, 162-172 (2015).

21. Kempermann, G. Environmental enrichment, new neurons and the neurobiology of individuality. Nat Rev Neurosci 20, 235-245 (2019).

22. McInnes, L., Healy, J. and Melville, J. UMAP: Uniform Manifold Approximation and Projection for Dimension Reduction. (2018).

23. Anderson, K. M. et al. Gene expression links functional networks across cortex and striatum. Nat Commun 9, (2018).

24. Zhang, J. and Jiao, J. Molecular Biomarkers for Embryonic and Adult Neural Stem Cell and Neurogenesis. Biomed Res Int 2015, 727542 (2015).

25. Valor, L. M., Charlesworth, P., Humphreys, L, Anderson, C. N. G. and Grant, S. G. N. Network activity-independent coordinated gene expression program for synapse assembly. www.pnas.orgcgidoil0.1073pnas. 0609071104 (2007).

26. Brunet, J. P., Tamayo, P., Golub, T. R. and Mesirov, J. P. Metagenes and molecular pattern discovery using matrix factorization. Proc Natl Acad Sei USA 101, 4164-4169 (2004).

27. Priness, I., Maimon, O. and Ben-Gal, I. Evaluation of gene-expression clustering via mutual information distance measure. BMC Bioinformatics 8, (2007).

28. van den Heuvel, M. P. and Sporns, O. Rich-club organization of the human connectome. Journal of Neuroscience 31, 15775-15786 (2011).

29. McAuley, J. J., Da Fontoura Costa, L. and Caetano, T. S. Rich-club phenomenon across complex network hierarchies. Appl Phys Lett 91, 2-5 (2007).

30. Watts, D. J. and Strogatz, S. H. Collective dynamics of'small-world' networks. Nature 393, 440-2 (1998).

31. Barabäsi, A.-L. and Albert, R. Emergence of Scaling in Random Networks. Science (1979) 286, 509-512 (1999).

32. Overall, R. W. and Kempermann, G. The Small World of Adult Hippocampal Neurogenesis. Front Neurosci 12, 1-12 (2018).

33. Arnatkeviciute, A. et al. Genetic influences on hub connectivity of the human connectome. Nat Commun 12, 1-14 (2021).

34. Zador, A. M. et al. Sequencing the Connectome. PLoS Biol 10, 1-7 (2012).

35. Haghverdi, L, Büttner, M., Wolf, F. A., Buettner, F. and Theis, F. J. Diffusion pseudotime robustly reconstructs lineage branching. Nat Methods 13, 845-848 (2016).

36. Zhang, W. and Linden, D. J. The other side of the engram: experience-driven changes in neuronal intrinsic excitability. Nat Rev Neurosci 4, 885-900 (2003).

37. Hübener, M. and Bonhoeffer, T. Searching for Engrams. Neuron 67, 363-371 (2010).

38. Gouwens, N. W. et al. Classification of electrophysiological and morphological neuron types in the mouse visual cortex. Nat Neurosci 22, 1182-1195 (2019).

39. Ma, Y. and Zhou, X. Spatially informed cell-type deconvolution for spatial transcriptomics. Nat Biotechnol (2022) doi:10.1038/s41587-022-01273-7.

40. Cable, D. M. et al. Robust decomposition of cell type mixtures in spatial transcriptomics. Nat Biotechnol 40, 517-526 (2022).

41. Erwin, S. R. et al. A Sparse, Spatially Biased Subtype of Mature Granule Cell Dominates Recruitment in Hippocampal-Associated Behaviors. Cell Rep 31, (2020).

42. Klausberger, T. and Somogyi, P. Neuronal diversity and temporal dynamics: The unity of hippocampal circuit operations. Science (1979) 321, 53-57 (2008).

43. Pignatelli, M. et al. Engram Cell Excitability State Determines the Efficacy of Memory Retrieval. Neuron 101, 274-284.e5 (2019).

44. Steinert, J. R., Chernova, T. and Forsythe, I. D. Nitric oxide signaling in brain function, dysfunction, and dementia. Neuroscientist 16, 435-452 (2010).

45. Bon, C. L. M. and Garthwaite, J. On the role of nitric oxide in hippocampal long-term potentiation. Journal of Neuroscience 23, 1941-1948 (2003).

46. Oberländer, K. et al. Dysregulation of Npas4 and Inhba expression and an altered excitation-inhibition balance are associated with cognitive deficits in DBA/2 mice. Learning and Memory 29, 55-70 (2022).

47. Li, W., Yin, Y., Quan, X. and Zhang, H. Gene Expression Value Prediction Based on XGBoost Algorithm. Front Genet 10, 1-7 (2019).

48. Asp, M., Bergensträhle, J. and Lundeberg, J. Spatially Resolved Transcriptomes- Next Generation Tools for Tissue Exploration. BioEssays 42, 1-16 (2020).

49. Moore, H., Lega, B. C. and Konopka, G. Riding brain "waves" to identify human memory genes. Curr Opin Cell Biol 78, 102118 (2022).

50. He, B. J. Scale-free brain activity: Past, present, and future. Trends Cogn Sei 18, 480-487 (2014).

51. Sejnowski, T. J., Churchland, P. S. and Movshon, J. A. Putting big data to good use in neuroscience. Nat Neurosci 17, 1440-1441 (2014).

52. Siegel, M., Donner, T. H. and Engel, A. K. Spectral fingerprints of large-scale neuronal interactions. Nat Rev Neurosci 13, 121-134 (2012).

53. Dean Ho, Stephen R. Quake, Edward R.B. McCabe, Wee Joo Chng, Edward K. Chow, Xianting Ding, Bruce D. Gelb, Geoffrey S. Ginsburg, Jason Hassenstab, Chih-Ming Ho, William C. Mobley, Garry P Nolan, Steven T. Rosen, A. Z. Enabling technologies for personalized and precision medicine. Trends Biotechnol 38, 497-518 (2019).

54. Berdondini, L. et al. Active pixel sensor array for high spatio-temporal resolution electrophysiological recordings from single cell to large scale neuronal networks. Lab Chip 9, 2644-51 (2009).

55. Heer, F. et al. Single-chip microelectronic system to interface with living cells. Biosens Bioelectron 22, 2546-53 (2007).

56. Jun, J. J. et al. Fully integrated silicon probes for high-density recording of neural activity. Nature 551, 232-236 (2017).

57. Angotzi, G. N. et al. SiNAPS: An implantable active pixel sensor CMOS-probe for simultaneous large-scale neural recordings. Biosens Bioelectron 126, 355-364 (2019).

58. Hao, Y. et al. Integrated analysis of multimodal single-cell data. Cell 184, 3573-3587. e29 (2021).

59. Korsunsky, I. et al. Fast, sensitive and accurate integration of single-cell data with Harmony. Nat Methods 16, 1289-1296 (2019).

60. Wolf, F. A., Angerer, P. and Theis, F. J. SCANPY: large-scale single-cell gene expression data analysis. Genome Biol 19, 15 (2018).

61. Anderson, K. M. et al. Gene expression links functional networks across cortex and striatum. Nat Commun 9, 1428 (2018).

62. Benjamini, Y. and Hochberg, Y. Controlling the False Discovery Rate: A Practical and Powerful Approach to Multiple Testing. Journal of the Royal Statistical Society: Series B (Methodological) 57, 289-300 (1995).

63. Pedregosa, F. et al. Scikit-learn: Machine Learning in Python. Journal of Machine Learning Research vol. 12 http://scikit-learn.sourceforge.net. (2011).

64. Barabäsi, A.-L. and Albert, R. Emergence of Scaling in Random Networks Downloaded from. Mat. Res. Soc. Symp. Proc vol. 74 www. sciencemag.org http://science. sciencemag.org/ (1995).

65. Virtanen, P. et al. SciPy 1.0: fundamental algorithms for scientific computing in Python. Nat Methods 17, 261-272 (2020).

66. Moon, K. R. et al. Visualizing structure and transitions in high-dimensional biological data. Nat Biotechnol 37, 1482-1492 (2019).

67. Chicco, D., Warrens, M. J. and Jurman, G. The coefficient of determination R-squared is more informative than SMAPE, MAE, MAPE, MSE and RMSE in regression analysis evaluation. PeerJ ComputSciT, 1-24 (2021).

## Claims

1. An *ex vivo* method for detecting the spatiotemporal electrophysiological dynamics and transcriptional profiles of functional neuronal cell assemblies comprising the steps of

   i) providing a functional neuronal cell assembly to be characterized,
   ii) recording patterns of local field potentials (LFPs) of the neuronal cells in the assembly at high spatial and temporal resolution using a suitable electrode array, and
   iii) obtaining spatial localization patterns of the LFPs of substantially the entire assembly using optical imaging, optionally, cryopreserving substantially the entire assembly,
   iv) performing optical imaging and spatially resolved transcriptomic analysis of the cells in the assembly using a second array, and
   v) processing the data as obtained comprising aligning the spatial localization patterns of the LFPs with the spatially resolved transcriptomic analysis, and
   thereby detecting the spatiotemporal electrophysiological dynamics and spatial transcriptional profiles of cells in the functional neuronal cell assembly,
   wherein preferably the recording of the patterns of local field potentials (LFPs) comprises the use of a high-density CMOS-based biosensing MEA chip (CMOS-MEA).

2. The method according to claim 1, wherein the functional neuronal cell assembly to be characterized is selected from a neuronal tissue, a brain slice, a sample of a neurological cancer, a culture of interconnected brain cells, cultured neuronal differentiated iPSCs, spinal marrow, electrogenic tissues like the olfactory bulb and cardiac tissues, and parts or combinations thereof, in particular a neuronal tissue derived from a mammal having a behavioral condition or disorder, a mammal having a neurological condition or disorder, such as a neurodegenerative disorder or cancer, and/or a mammal being treated for one of these conditions or disorders.

3.  The method according to claim 1 or 2, wherein the optical imaging comprises imaging of the entire network of cell assemblies, for example using bright-filed imaging, comprising the use of a microscope, such as a modular stereomicroscope.

4.  The method according to any one of claims 1 to 3, wherein the recording of the LFPs and the optical imaging is performed simultaneously.

5.  The method according to any one of claims 1 to 4, wherein cryopreserving comprises rapid freezing, preferably over dry ice, and optionally further comprises storing at -80°C.

6.  The method according to any one of claims 1 to 5, wherein the cell assemblies are suitably stained before the transcriptomic analysis.

7.  The method according to any one of claims 1 to 6, wherein the transcriptomics comprises the generation of barcode-labeled cDNA and sequencing, such as quantitative sequencing, on the second array, such as, for example, spatially-resolved transcriptomics (SRT) sequencing.

8.  The method according to any one of claims 1 to 7, wherein the assessment and allocation of the recorded LFPs to specific features and shapes of waveforms from interconnected network layers comprises a grouping of the electrodes and the use of principal component analysis (PCA) and K-means clustering algorithms.

9.  The method according to any one of claims 1 to 8, wherein processing the data and aligning the spatial localization patterns of the LFPs with the spatially resolved sequencing and transcriptomic analysis, comprises overlaying the optical images with the electrode array layout, and overlaying the optical images with the second array layout, and alignment of the two overlays comprising image resizing and rotation.

10. A method for identifying the composition of functional neuronal assemblies, comprising performing the method according to any one of claims 1 to 9, and further comprising the step of concluding on the composition of the functional neuronal cell assemblies based on the spatiotemporal electrophysiological dynamics and transcriptional profiles of the cells as detected, wherein preferably the identifying comprises mapping of the cells in the assembly, the identification of clusters, such as functional clusters, of the cells in the assembly, the identification of diverse neural cell types in the assembly, and/or the identification of spectral characteristics of cells in the assembly.

11. A method for monitoring the spatiotemporal electrophysiological dynamics and transcriptional profiles of functional neuronal cell assemblies comprising performing the method according to any one of claims 1 to 9 repeatedly at different time points, and monitoring the spatial electrophysiological and transcriptional information of the molecular neuronal cell assemblies based on comparing the spatiotemporal electrophysiological dynamics and transcriptional profiles of the cells as detected at different timepoints.

12. A method for monitoring the cellular composition of a functional neuronal cell assembly, comprising performing the method according to claim 10 repeatedly at different time points, and monitoring the cellular composition of the functional neuronal cell assembly based on comparing the cellular compositions of the functional neuronal cell assembly as detected at different timepoints.

13. A method for identifying a compound having an effect on the spatiotemporal electrophysiological and transcriptional profile and/or composition of cells in a functional neuronal cell assembly comprising performing the method according to any one of claims 1 to 10 in the presence and absence of at least one candidate compound, wherein a difference of the spatiotemporal electrophysiological and transcriptional dynamics and/or composition of cells in the functional neuronal cell assembly in the presence and absence of the at least one candidate compound or when compared to a suitable control identifies a compound having an effect on the spatiotemporal electrophysiological dynamics and transcriptional profile and/or composition of cells in a functional neuronal cell assembly, wherein preferably the effect is a therapeutic effect, such as a modification of the cellular composition, changes in gene expression, an anti-cancer effect, a neuroprotective effect, a morphological effect, a cognitive effect and/or a behavioral effect.

14. A device for performing the method according to any one of claims 1 to 13, in particular comprising the neuronal cell assembly to be analyzed, an electrode array suitable to record patterns of local field potentials (LFPs) of the assembly while performing optical imaging, a second array suitable to perform spatially resolved transcriptomic analysis of the cells in the assembly while performing optical imaging, and a computer program product for processing the data as obtained comprising aligning the spatial localization patterns of the LFPs with the spatially resolved sequencing and transcriptomic analysis, optionally with additional buffers, and instructions for use.

15. Use of the device according to claim 14 for detecting the spatiotemporal electrophysiological dynamics and transcriptional profile of cells in a functional neuronal cell assembly, for identifying the composition of a functional neuronal cell assembly, for monitoring the spatial electrophysiological and transcriptional dynamics of cells in a functional neuronal cell assembly, for monitoring the cellular composition of a functional neuronal cell assembly, or for identifying a compound having an effect on the spatiotemporal electrophysiological dynamics and transcriptional profile and/or for identifying the composition of cells in a functional neuronal cell assembly.

Figure 1

Figure 2

**a**

EP 4 560 314 A1

Figure 2 (continued)

**b**

## SRT Correlation with network-wide LFP Rate

SD

ENR

Total Genes: 19383

Total Genes: 19795

DG    CA3    CA1

SD

ENR

EC

DG

Hilus    CA1

CA3    PC

DG  CA3  CA1
$0.05 \leq p_{adj} < 0.1$
$0.01 < p_{adj} < 0.05$
$p_{adj} \leq 0.01$
uncorrelated genes

ENR Fold Change Increase
DG: 2.27    CA3: 2.54    CA1: 0.60

**c**

V-Matrix
(Multimodal Multiscale data features)

$\approx$

W-Matrix
(Spatiotemporal distribution
of factor weights)

$\times$

H-Matrix
(Spatiotemporal pattern factorization)

EP 4 560 314 A1

Figure 2 (continued)

Figure 2 (continued)

Figure 3

Figure 3 (continued)

Figure 4

Figure 4 (continued)

**c** SD DPT-CAT

**d** ENR DPT-CAT

Figure 4 (continued)

EP 4 560 314 A1

Figure 5

Figure 5 (continued)

Figure 5 (continued)

Figure 6

EP 4 560 314 A1

Figure 6 (continued)

EP 4 560 314 A1

Figure 7

Figure 7 (continued)

e

f

EP 4 560 314 A1

Figure 8

EP 4 560 314 A1

Figure 8 (continued)

b

d

f

g

EP 4 560 314 A1

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2022/093940 A1 (BROAD INST INC [US]; MASSACHUSETTS INST TECHNOLOGY ET AL.) 5 May 2022 (2022-05-05) * paragraph [0006] - paragraph [0048]; figures 3A, 4A, 4I, 10, 12A, 13A, 13C, 18A-18C, 25C * * paragraph [0086] - paragraph [0122] * | 1-15 | INV. G01N33/483 G01N21/00 |
| A | Waka Lin et al.: "Dendritic spine formation and synapse maturation in transcription factor-induced human iPSC-derived neurons", iScience, 21 April 2023 (2023-04-21), pages 1-22, XP093097683, DOI: doi.org/10.1016/j.isci.2023.106285 Retrieved from the Internet: URL:https://www.sciencedirect.com/science/article/pii/S2589004223003620/pdfft?md5=2aa73780fa774843231f83743cb78901&pid=1-s2.0-S2589004223003620-main.pdf [retrieved on 2023-11-02] * the whole document * | 1-15 | |

TECHNICAL FIELDS
SEARCHED    (IPC)

G01N

The present search report has been drawn up for all claims

| Place of search | Date of completion of the search | Examiner |
|---|---|---|
| The Hague | 3 July 2024 | Stavroulakis, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 21 2407

This annex lists the patent family members relating to the patent documents cited in the above-mentioned European search report.
The members are as contained in the European Patent Office EDP file on
The European Patent Office is in no way liable for these particulars which are merely given for the purpose of information.

03-07-2024

| Patent document cited in search report | Publication date | Patent family member(s) | Publication date |
|---|---|---|---|
| WO 2022093940 A1 | 05-05-2022 | US 2024019353 A1 | 18-01-2024 |
| | | WO 2022093940 A1 | 05-05-2022 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2023091970 A1
- US 20220068438 A1
- WO 2021168455 A1

### Non-patent literature cited in the description

- **AQRAWE Z et al.** A simultaneous optical and electrical in-vitro neuronal recording system to evaluate microelectrode performance. *PLoS One*, 20 August 2020, vol. 15 (8), e0237709
- **HOLTMAAT, A.** ; **CARONI, P.** Functional and structural underpinnings of neuronal assembly formation in learning. *Nat Neurosci*, 2016, vol. 19, 1553-1562, https://doi.org/10.1038/nn.4418
- **UMBACH, G.** ; **TAN, R.** ; **JACOBS, J. et al.** Flexibility of functional neuronal assemblies supports human memory. *Nat Commun*, 2022, vol. 13, 6162, https://doi.org/10.1038/s41467-022-33587-0
- **HU X** ; **KHANZADA S** ; **KLÜTSCH D** ; **CALEGARI F** ; **AMIN H**. Implementation of biohybrid olfactory bulb on a high-density CMOS-chip to reveal large-scale spatiotemporal circuit information. *Biosens Bioelectron.*, 24 November 2021, vol. 198, 113834
- **EMERY BA** ; **HU X** ; **KHANZADA S** ; **KEMPERMANN G** ; **AMIN H**. High-resolution CMOS-based biosensor for assessing hippocampal circuit dynamics in experience-dependent plasticity. *Biosens Bioelectron.*, 12 June 2023, vol. 237, 115471
- **DUAN H** ; **CHENG T** ; **CHENG H**. Spatially resolved transcriptomics: advances and applications. *Blood Sci.*, 04 November 2022, vol. 5 (1), 1-14
- **WANG X** ; **ALMET AA** ; **NIE Q**. The promising application of cell-cell interaction analysis in cancer from single-cell and spatial transcriptomics. *Semin Cancer Biol.*, 15 July 2023, vol. 95, 42-51
- **FRACKOWIAK, R.** ; **MARKRAM, H.** The future of human cerebral cartography: A novel approach. *Philosophical Transactions of the Royal Society B: Biological Sciences*, 2015, vol. 370
- **PARENT!, I.** ; **RABANEDA, L. G.** ; **SCHOEN, H.** ; **NOVARINO, G.** Neurodevelopmental Disorders: From Genetics to Functional Pathways. *Trends Neurosci*, 2020, vol. 43, 608-621
- **PALOP, J. J.** ; **MUCKE, L.** Network abnormalities and interneuron dysfunction in Alzheimer disease. *Nat Rev Neurosci*, 2016, vol. 17, 777-792

- **CADWELL, C. R. et al.** Electrophysiological, transcriptomic and morphologic profiling of single neurons using Patch-seq. *Nat Biotechnol*, 2016, vol. 34, 199-203
- **FUZIK, J. et al.** Integration of electrophysiological recordings with single-cell RNA-seq data identifies neuronal subtypes. *Nat Biotechnol*, 2016, vol. 34, 175-183
- **LI, Q. et al.** Multimodal charting of molecular and functional cell states via in situ electro-sequencing. *Cell*, 2023, vol. 186, 2002-2017.e21
- **STÄHL, P. L. et al.** Visualization and analysis of gene expression in tissue sections by spatial transcriptomics. *Science*, 1979, vol. 353, 78-82
- **VICKOVIC, S. et al.** High-definition spatial transcriptomics for in situ tissue profiling. *Nat Methods*, 2020, vol. 16, 987-990
- **MANIATIS, S. et al.** Spatiotemporal dynamics of molecular pathology in amyotrophic lateral sclerosis. *Science*, 1979, vol. 364, 89-93
- **ASP, M. et al.** A Spatiotemporal Organ-Wide Gene Expression and Cell Atlas of the Developing Human Heart. *Cell*, 2019, vol. 179, 1647-1660.el9
- **URAI, A. E.** ; **DOIRON, B.** ; **LEIFER, A. M.** ; **CHURCHLAND, A. K.** Large-scale neural recordings call for new insights to link brain and behavior. *Nat Neurosci*, 2021, vol. 25
- **BUZSÄKI, G.** Large-scale recording of neuronal ensembles. *Nat Neurosci*, 2004, vol. 7, 446-451
- **HU, X.** ; **KHANZADA, S.** ; **KLÜTSCH, D.** ; **CALEGARI, F.** ; **AMIN, H.** Implementation of biohybrid olfactory bulb on a high-density CMOS-chip to reveal large-scale spatiotemporal circuit information. *Biosens Bioelectron*, 2022, vol. 198, 113834
- **AMIN, H.** ; **NIEUS, T.** ; **LONARDONI, D.** ; **MACCIONE, A.** ; **BERDONDINI, L.** *Sei. Rep.*, 2017, vol. 2460, 7
- **IMFELD, K. et al.** Large-scale, high-resolution data acquisition system for extracellular recording of electrophysiological activity. *IEEE Trans Biomed Eng*, 2008, vol. 55, 2064-73

- **EMERY, B. A.** ; **HU, X.** ; **KHANZADA, S.** ; **KEMPERMANN, G.** ; **AMIN, H.** High-resolution CMOS-based biosensor for assessing hippocampal circuit dynamics in experience-dependent plasticity. *Biosens Bioelectron*, 2023, 115471
- **EMERY, B. A. et al.** Large-scale Multimodal Neural Recordings on a High-density Neurochip: Olfactory Bulb and Hippocampal Networks. *Ieee Embs*, 2022, 42-45
- **LUO, L.** ; **CALLAWAY, E. M.** ; **SVOBODA, K.** Genetic Dissection of Neural Circuits: A Decade of Progress. *Neuron*, 2018, vol. 98, 256-281
- **PANZERI, S.** ; **MACKE, J. H.** ; **GROSS, J.** ; **KAYSER, C.** Neural population coding: Combining insights from microscopic and mass signals. *Trends Cogn Sei*, 2015, vol. 19, 162-172
- **KEMPERMANN, G.** Environmental enrichment, new neurons and the neurobiology of individuality. *Nat Rev Neurosci*, 2019, vol. 20, 235-245
- **MCLNNES, L.** ; **HEALY, J.** ; **MELVILLE, J.** *UMAP: Uniform Manifold Approximation and Projection for Dimension Reduction*, 2018
- **ANDERSON, K. M. et al.** Gene expression links functional networks across cortex and striatum. *Nat Commun*, 2018, vol. 9
- **ZHANG, J.** ; **JIAO, J.** Molecular Biomarkers for Embryonic and Adult Neural Stem Cell and Neurogenesis. *Biomed Res Int*, 2015, vol. 2015, 727542
- **VALOR, L. M.** ; **CHARLESWORTH, P.** ; **HUMPHREYS, L** ; **ANDERSON, C. N. G.** ; **GRANT, S. G. N.** *Network activity-independent coordinated gene expression program for synapse assembly*, 2007
- **BRUNET, J. P.** ; **TAMAYO, P.** ; **GOLUB, T. R.** ; **MESIROV, J. P.** Metagenes and molecular pattern discovery using matrix factorization. *Proc Natl Acad Sei USA*, 2004, vol. 101, 4164-4169
- **PRINESS, I.** ; **MAIMON, O.** ; **BEN-GAL, I.** Evaluation of gene-expression clustering via mutual information distance measure. *BMC Bioinformatics*, 2007, vol. 8
- **VAN DEN HEUVEL, M. P.** ; **SPORNS, O.** Rich-club organization of the human connectome. *Journal of Neuroscience*, 2011, vol. 31, 15775-15786
- **MCAULEY, J. J.** ; **DA FONTOURA COSTA, L.** ; **CAETANO, T. S.** Rich-club phenomenon across complex network hierarchies. *Appl Phys Lett*, 2007, vol. 91, 2-5
- **WATTS, D. J.** ; **STROGATZ, S. H.** Collective dynamics of 'small-world' networks. *Nature*, 1998, vol. 393, 440-2
- **BARABÄSI, A.-L.** ; **ALBERT, R.** Emergence of Scaling in Random Networks. *Science*, 1979, vol. 286, 509-512
- **OVERALL, R. W.** ; **KEMPERMANN, G.** The Small World of Adult Hippocampal Neurogenesis. *Front Neurosci*, 2018, vol. 12, 1-12
- **ARNATKEVICIUTE, A. et al.** Genetic influences on hub connectivity of the human connectome. *Nat Commun*, 2021, vol. 12, 1-14
- **ZADOR, A. M. et al.** Sequencing the Connectome. *PLoS Biol*, 2012, vol. 10, 1-7
- **HAGHVERDI, L** ; **BÜTTNER, M.** ; **WOLF, F. A.** ; **BUETTNER, F.** ; **THEIS, F. J.** Diffusion pseudotime robustly reconstructs lineage branching. *Nat Methods*, 2016, vol. 13, 845-848
- **ZHANG, W.** ; **LINDEN, D. J.** The other side of the engram: experience-driven changes in neuronal intrinsic excitability. *Nat Rev Neurosci*, 2003, vol. 4, 885-900
- **HÜBENER, M.** ; **BONHOEFFER, T.** Searching for Engrams. *Neuron*, 2010, vol. 67, 363-371
- **GOUWENS, N. W. et al.** Classification of electrophysiological and morphological neuron types in the mouse visual cortex. *Nat Neurosci*, 2019, vol. 22, 1182-1195
- **MA, Y.** ; **ZHOU, X.** Spatially informed cell-type deconvolution for spatial transcriptomics. *Nat Biotechnol*, 2022
- **CABLE, D. M. et al.** Robust decomposition of cell type mixtures in spatial transcriptomics. *Nat Biotechnol*, 2022, vol. 40, 517-526
- **ERWIN, S. R. et al.** A Sparse, Spatially Biased Subtype of Mature Granule Cell Dominates Recruitment in Hippocampal-Associated Behaviors. *Cell Rep*, 2020, vol. 31
- **KLAUSBERGER, T.** ; **SOMOGYI, P.** Neuronal diversity and temporal dynamics: The unity of hippocampal circuit operations. *Science*, 1979, vol. 321, 53-57
- **PIGNATELLI, M. et al.** Engram Cell Excitability State Determines the Efficacy of Memory Retrieval. *Neuron*, 2019, vol. 101, 274-284.e5
- **STEINERT, J. R.** ; **CHERNOVA, T.** ; **FORSYTHE, I. D.** Nitric oxide signaling in brain function, dysfunction, and dementia. *Neuroscientist*, 2010, vol. 16, 435-452
- **BON, C. L. M.** ; **GARTHWAITE, J.** On the role of nitric oxide in hippocampal long-term potentiation. *Journal of Neuroscience*, 2003, vol. 23, 1941-1948
- **OBERLÄNDER, K. et al.** Dysregulation of Npas4 and Inhba expression and an altered excitation-inhibition balance are associated with cognitive deficits in DBA/2 mice. *Learning and Memory*, 2022, vol. 29, 55-70
- **LI, W.** ; **YIN, Y.** ; **QUAN, X.** ; **ZHANG, H.** Gene Expression Value Prediction Based on XGBoost Algorithm. *Front Genet*, 2019, vol. 10, 1-7
- **ASP, M.** ; **BERGENSTRÄHLE, J.** ; **LUNDEBERG, J.** Spatially Resolved Transcriptomes- Next Generation Tools for Tissue Exploration. *BioEssays*, 2020, vol. 42, 1-16
- **MOORE, H.** ; **LEGA, B. C.** ; **KONOPKA, G.** Riding brain "waves" to identify human memory genes. *Curr Opin Cell Biol*, 2022, vol. 78, 102118

- **HE, B. J.** Scale-free brain activity: Past, present, and future. *Trends Cogn Sei*, 2014, vol. 18, 480-487
- **SEJNOWSKI, T. J.** ; **CHURCHLAND, P. S.** ; **MOV-SHON, J. A.** Putting big data to good use in neuroscience. *Nat Neurosci*, 2014, vol. 17, 1440-1441
- **SIEGEL, M.** ; **DONNER, T. H.** ; **ENGEL, A. K.** Spectral fingerprints of large-scale neuronal interactions. *Nat Rev Neurosci*, 2012, vol. 13, 121-134
- **DEAN HO** ; **STEPHEN R. QUAKE** ; **EDWARD R.B. MCCABE** ; **WEE JOO CHNG** ; **EDWARD K. CHOW** ; **XIANTING DING** ; **BRUCE D. GELB** ; **GEOFFREY S. GINSBURG** ; **JASON HASSENSTAB** ; **CHIH-MING HO**. A. Z. Enabling technologies for personalized and precision medicine. *Trends Biotechnol*, 2019, vol. 38, 497-518
- **BERDONDINI, L. et al.** Active pixel sensor array for high spatio-temporal resolution electrophysiological recordings from single cell to large scale neuronal networks. *Lab Chip*, 2009, vol. 9, 2644-51
- **HEER, F. et al.** Single-chip microelectronic system to interface with living cells. *Biosens Bioelectron*, 2007, vol. 22, 2546-53
- **JUN, J. J. et al.** Fully integrated silicon probes for high-density recording of neural activity. *Nature*, 2017, vol. 551, 232-236
- **ANGOTZI, G. N. et al.** SiNAPS: An implantable active pixel sensor CMOS-probe for simultaneous large-scale neural recordings. *Biosens Bioelectron*, 2019, vol. 126, 355-364
- **HAO, Y. et al.** Integrated analysis of multimodal single-cell data. *Cell*, 2021, vol. 184, 3573-3587
- **KORSUNSKY, I. et al.** Fast, sensitive and accurate integration of single-cell data with Harmony. *Nat Methods*, 2019, vol. 16, 1289-1296
- **WOLF, F. A.** ; **ANGERER, P.** ; **THEIS, F. J.** SCANPY: large-scale single-cell gene expression data analysis. *Genome Biol*, 2018, vol. 19, 15
- **ANDERSON, K. M. et al.** Gene expression links functional networks across cortex and striatum. *Nat Commun*, 2018, vol. 9, 1428
- **BENJAMINI, Y.** ; **HOCHBERG, Y.** Controlling the False Discovery Rate: A Practical and Powerful Approach to Multiple Testing. *Journal of the Royal Statistical Society: Series B (Methodological)*, 1995, vol. 57, 289-300
- **PEDREGOSA, F. et al.** Scikit-learn: Machine Learning in Python. *Journal of Machine Learning Research*, 2011, vol. 12, http://scikit-learn.sourceforge.net
- **BARABÄSI, A.-L.** ; **ALBERT, R.** Emergence of Scaling in Random Networks Downloaded from. *Mat. Res. Soc. Symp. Proc*, 1995, vol. 74, www. sciencemag.org http://science. sciencemag.org
- **VIRTANEN, P. et al.** SciPy 1.0: fundamental algorithms for scientific computing in Python. *Nat Methods*, 2020, vol. 17, 261-272
- **MOON, K. R. et al.** Visualizing structure and transitions in high-dimensional biological data. *Nat Biotechnol*, 2019, vol. 37, 1482-1492
- **CHICCO, D.** ; **WARRENS, M. J.** ; **JURMAN, G.** The coefficient of determination R-squared is more informative than SMAPE, MAE, MAPE, MSE and RMSE in regression analysis evaluation. *PeerJ ComputSciT*, 2021, 1-24